(19) Europäisches Patentamt / European Patent Office / Office européen des brevets

(11) **EP 3 796 108 B1**

(12) **EUROPÄISCHE PATENTSCHRIFT**

(45) Veröffentlichungstag und Bekanntmachung des Hinweises auf die Patenterteilung:
**08.05.2024 Patentblatt 2024/19**

(21) Anmeldenummer: **19198558.9**

(22) Anmeldetag: **20.09.2019**

(51) Internationale Patentklassifikation (IPC):
**G05B 13/02** (2006.01)  **G06N 5/00** (2023.01)
**G06N 3/04** (2023.01)  **G06N 7/00** (2023.01)
**G06N 20/20** (2019.01)  **G06N 7/01** (2023.01)
**G06N 5/01** (2023.01)

(52) Gemeinsame Patentklassifikation (CPC):
**G05B 13/021; G06N 5/01; G06N 7/01; G06N 20/20;**
G06N 3/047; G06N 20/10

(54) **VORRICHTUNG UND VERFAHREN ZUM ERMITTELN EINES ROBUSTEN OPTIMUMS EINES PHYSIKALISCHEN ODER CHEMISCHEN PROZESSES GEMÄSS EINEM BAYES OPTIMIERUNGSVERFAHREN**

DEVICE AND METHOD FOR DETERMINING A ROBUST OPTIMUM OF A PHYSICAL OR CHEMICAL PROCESS ACCORDING TO A BAYESIAN OPTIMISATION METHOD

DISPOSITIF ET PROCÉDÉ DE DÉTERMINATION D'UN OPTIMUM SOLIDE D'UN PROCESSUS PHYSIQUE OU CHIMIQUE SELON UN PROCÉDÉ D'OPTIMISATION DE BAYES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**24.03.2021 Patentblatt 2021/12**

(73) Patentinhaber: **Robert Bosch GmbH**
**70442 Stuttgart (DE)**

(72) Erfinder:
• **Klenske, Edgar**
 **71272 Renningen (DE)**
• **Froehlich, Lukas**
 **70469 Stuttgart (DE)**
• **Vinogradska, Julia**
 **70469 Stuttgart (DE)**
• **Zeilinger, Melanie**
 **8092 Zürich (CH)**

(56) Entgegenhaltungen:
**US-A1- 2007 129 930**

• **SHION TAKENO ET AL: "Multi-fidelity Bayesian Optimization with Max-value Entropy Search and its parallelization", ARXIV.ORG, CORNELL UNIVERSITY LIBRARY, 201 OLIN LIBRARY CORNELL UNIVERSITY ITHACA, NY 14853, 24. Januar 2019 (2019-01-24), XP081598314,**
• **ZI WANG ET AL: "Max-value Entropy Search for Efficient Bayesian Optimization", ARXIV.ORG, CORNELL UNIVERSITY LIBRARY, 201 OLIN LIBRARY CORNELL UNIVERSITY ITHACA, NY 14853, 6. März 2017 (2017-03-06), XP081305451,**
• **GARRIDO-MERCHÁN EDUARDO C ET AL: "Predictive Entropy Search for Multi-objective Bayesian Optimization with Constraints", NEUROCOMPUTING, ELSEVIER, AMSTERDAM, NL, Bd. 361, 13. Juni 2019 (2019-06-13), Seiten 50-68, XP085797175, ISSN: 0925-2312, DOI: 10.1016/J.NEUCOM.2019.06.025 [gefunden am 2019-06-13]**
• **CALANDRA ROBERTO ET AL: "Bayesian optimization for learning gaits under uncertainty", ANNALS OF MATHEMATICS AND ARTIFICIAL INTELLIGENCE, BALTZER, BASEL, CH, Bd. 76, Nr. 1, 26. Juni 2015 (2015-06-26), Seiten 5-23, XP035899004, ISSN: 1012-2443, DOI: 10.1007/S10472-015-9463-9 [gefunden am 2015-06-26]**

## Beschreibung

Verschiedene Ausführungsbeispiele betreffen allgemein eine Vorrichtung und ein Verfahren zum Ermitteln eines robusten Optimums eines physikalischen oder chemischen Prozesses gemäß einem *Bayes-Optimierungsverfahren.*

Bei Produktionsprozessen und Bearbeitungsprozessen werden Prozessparameter, wie beispielsweise Prozesstemperatur, Prozesszeit, ein Vakuum bzw. eine Gasatmosphäre usw., derart eingestellt, um gewünschte Eigenschaften, wie beispielsweise Härte, Festigkeit, Wärmeleitfähigkeit, elektrische Leitfähigkeit usw., eines Werkstücks zu erhalten. Die Prozessparameter können durch modellbasierte Optimierungsverfahren, wie beispielsweise Bayes-Optimierungsverfahren, ermittelt werden. Allerdings können die Prozessparameter Rauschen oder Störungen unterliegen, sodass die Prozessparameter von den durch das Optimierungsverfahren ermittelten Prozessparametern abweichen können und damit die Eigenschaften des Werkstücks von den gewünschten Eigenschaften abweichen können. Daher ist es zum Beispiel bei Produktionsprozessen und Bearbeitungsprozessen erforderlich, ein robustes Optimum zu ermitteln, in dem das Werkstück die gewünschten Eigenschaften aufweist.

Verschiedene Modelle können verwendet werden, um bei verschiedenen physikalischen oder chemischen Prozessen Prozessparameter und/oder prozessrelevante Eigenschaften von Materialien, wie beispielsweise Härte, elektrische Leitfähigkeit, Dichte, Mikrostruktur, Makrostruktur, chemische Zusammensetzung usw., zu ermitteln, um gewünschte Prozessergebnisse zu erhalten, wobei die Prozessparameter bzw. prozessrelevanten Eigenschaften von den Werten abweichen können, für die der jeweilige Prozess eingestellt (zum Beispiel optimiert) wurde. Daher kann es erforderlich sein, ein Modell bereitzustellen, das imstande ist, ein robustes Optimum zu ermitteln, bei dem diese Abweichungen berücksichtigt werden.

In Nogueira et al., Unscented Bayesian Optimization for Safe Robot Grasping, arXiv:1603.02038, 2016, ist ein Verfahren zum Ermitteln eines robusten Optimums beschrieben, wobei ein Robustheitskriterium unter Verwendung eines Erwartungswertes bezüglich eines Rauschens ermittelt wird.

In Beland et al., Bayesian Optimization Under Uncertainty, Conference on Neural Information Processing Systems, 2017, ist ein Verfahren zum Ermitteln eines robusten Optimums beschrieben, wobei ein Robustheitskriterium durch eine Gauß-Prozess-Regression ermittelt wird.

In Bogunovic et al., Adversarially Bayesian Optimization with Gaussian Processes, Conference on Neural Information Processing Systems, 2018, ist ein Verfahren zum Ermitteln eines robusten Optimums beschrieben.

In Takeno S. et al., Multi-fidelity Bayesian Optimization with Max-value Entropy Search and its parallelization, arXiv.org:1901.08275v2, 2019, wird ein parallel ausführbarer Ansatz basierend auf einer Suche nach der maximalen Entropie offenbart, wobei eine "Multi-fidelity Bayesian Optimization" verwendet wird.

Ein ähnliches Verfahren wird auch in Wang, Zi et al., Max-Value Entropy Search for Efficient Bayesian Optimization, arXiv.org:1703.01968v3, 2017, beschrieben.

Die Erfindung wird durch den beigefügten Anspruchssatz definiert.

Das Verfahren und die Vorrichtung mit den Merkmalen der unabhängigen Ansprüche 1 (erstes Beispiel) und 5 (vierundzwanzigstes Beispiel) ermöglichen, ein Prozessfenster durch ein Modell in Abhängigkeit von Abweichungen der Eingangsparameter zu ermitteln.

Ein statistisches Modell kann jede Art von mathematischer Darstellung sein, die einen Zusammenhang von Eingangsparametern und Ausgangsparametern eines physikalischen oder chemischen Prozesses beschreibt und die Wahrscheinlichkeitsverteilungen betrachtet.

Das Verfahren weist das Messen des dem Eingangsparameterwert des neuen Messpunktes zugeordneten Ausgangsparameterwertes auf. Das Verfahren weist das Adaptieren des ersten statistischen Modells unter Verwendung des neuen Messpunktes auf, wobei das adaptierte erste statistische Modell den physikalischen oder chemischen Prozess zu den bekannten Messpunkten und dem neuen Messpunkt beschreiben kann. Das Verfahren weist ferner das Ermitteln eines robusten Optimums des adaptierten ersten statistischen Modells auf. Das hat den Vorteil, dass ein oder mehrere Prozessparameter ermittelt werden können, für die der physikalische oder chemische Prozess robust gegenüber Störungen oder Rauschen der Prozessparameter ist.

Das Ermitteln eines robusten Optimums des ersten statistischen Modells weist das Ermitteln eines adaptierten zweiten statistischen Modells für das adaptierte erste statistische Modell und das Ermitteln eines globalen Maximums des adaptierten zweiten statistischen Modells auf, wobei der Eingangsparameterwert des globalen Maximums des zweiten statistischen Modells dem Eingangsparameterwert des robusten Optimums des ersten statistischen Modells entspricht.

Das Verfahren weist ferner das Steuern des physikalischen oder chemischen Prozesses auf, wobei der Eingangsparameterwert des physikalischen oder chemischen Prozesses in Abhängigkeit von dem Eingangsparameterwert des robusten Optimums gewählt wird, insbesondere diesem entspricht. Die in Anspruch 1 beschriebenen Merkmale werden auch als viertes Beispiel bezeichnet (das erste bis dritte Beispiel entfallen).

Das Verfahren kann ferner das Ermitteln eines Prozessfensters des physikalischen oder chemischen Prozesses unter Verwendung des robusten Optimums und der Veränderung der Eingangsparameterwerte aufweisen. Das hat den Vorteil, dass Prozessbereich ermittelt werden können, in dem die Prozessparameter des physikalischen oder chemischen Prozesses zu den gewünschten Eigenschaften bzw. einem gewünschten Erfolg des physikalischen oder chemischen Prozesses führen.

Der einem Eingangsparameterwert zugeordnete Eingangsparameter kann eine erste physikalische Größe sein. Der einem Ausgangsparameterwert zugeordnete Ausgangsparameter kann eine zweite physikalische Größe sein. Die in diesem Absatz beschriebenen Merkmale in Kombination mit dem vierten Beispiel bilden ein fünftes Beispiel.

Der Eingangsparameterwert eines Messpunktes der bekannten Messpunkte kann von einem ersten Sensor detektiert werden. Der Ausgangsparameterwert eines Messpunktes der bekannten Messpunkte kann von einem zweiten Sensor detektiert werden. Der Eingangsparameterwert des neuen Messpunktes kann von dem ersten Sensor detektiert werden und der Ausgangsparameterwert des neuen Messpunktes kann von dem zweiten Sensor detektiert werden. Die in diesem Absatz beschriebenen Merkmale in Kombination mit einem des zweiten Beispiels bis dem fünften Beispiel bilden ein sechstes Beispiel. Das erste statistische Modell kann mittels einer Gauß-Prozess-Regression unter Verwendung der bekannten Messpunkte ermittelt werden. Das in diesem Absatz beschriebene Merkmal in Kombination mit einem des vierten Beispiels bis dem sechsten Beispiel bildet ein siebtes Beispiel.

Das erste statistische Modell kann von einem bayesschen neuronalen Netzwerk gebildet werden. Das in diesem Absatz beschriebene Merkmal in Kombination mit einem des vierten Beispiels bis dem siebten Beispiel bildet ein achtes Beispiel.

Das zweite statistische Modell kann für das erste statistische Modell unter Verwendung einer Verteilung der Veränderung der Eingangsparameterwerte ermittelt werden. Das in diesem Absatz beschriebene Merkmal in Kombination mit einem des ersten Beispiels bis dem achten Beispiel bildet ein neuntes Beispiel.

Die Verteilung der Veränderung der Eingangsparameterwerte kann unter Verwendung der Varianz der Eingangsparameterwerte eines jeweiligen Messpunktes der bekannten Messpunkte ermittelt werden. Das in diesem Absatz beschriebene Merkmal in Kombination mit dem neunten Beispiel bildet ein zehntes Beispiel.

Eine Entropie des durch die bekannten Messpunkte beschriebenen ersten statistischen Modells kann unter Verwendung einer ersten prädiktiven Verteilung ermittelt werden. Das in diesem Absatz beschriebene Merkmal in Kombination mit einem des ersten Beispiels bis dem zehnten Beispiel bildet ein elftes Beispiel.

Die erste prädiktive Verteilung kann eine erste bedingte Wahrscheinlichkeitsverteilung des ersten statistischen Modells mit der Konditionierung auf die bekannten Messpunkte sein. Das in diesem Absatz beschriebene Merkmal in Kombination mit dem elften Beispiel bildet ein zwölftes Beispiel.

Die erste prädiktive Verteilung kann eine Gaußverteilung sein. Das in diesem Absatz beschriebene Merkmal in Kombination mit dem elften Beispiel oder dem zwölften Beispiel bildet ein dreizehntes Beispiel.

Die erste prädiktive Verteilung kann mittels eines Gauß-Prozesses ermittelt werden. Das in diesem Absatz beschriebene Merkmal in Kombination mit einem des elften Beispiels bis dem dreizehnten Beispiel bildet ein vierzehntes Beispiel.

Die erwartete Entropie kann unter Verwendung einer zweiten prädiktiven Verteilung ermittelt werden. Die zweite prädiktive Verteilung kann eine zweite bedingte Wahrscheinlichkeitsverteilung des ersten statistischen Modells mit der Konditionierung auf die bekannten Messpunkte und auf den angenommenen Maximalwert des Ausgangsparameters des zweiten statistischen Modells an dem robusten Optimum sein. Die in diesem Absatz beschriebenen Merkmale in Kombination mit einem des ersten Beispiels bis dem vierzehnten Beispiel bildet ein fünfzehntes Beispiel.

Die erwartete Entropie kann durch eine Monte-Carlo-Approximation ermittelt werden. Das in diesem Absatz beschriebene Merkmal in Kombination mit dem fünfzehnten Beispiel bildet ein sechzehntes Beispiel.

Die erwartete Entropie kann unter Verwendung einer Entropie des durch die bekannten Messpunkte beschriebenen ersten statistischen Modells und mehreren approximierten Maximalwerten des Ausgangsparameters des zweiten statistischen Modells an dem robusten Optimum des physikalischen oder chemischen Prozesses ermittelt werden. Das in diesem Absatz beschriebene Merkmal in Kombination mit dem sechzehnten Beispiel bildet ein siebzehntes Beispiel.

Die Entropie des durch die bekannten Messpunkte beschriebenen ersten statistischen Modells und mehreren approximierten Maximalwerten des Ausgangsparameters des zweiten statistischen Modells an dem robusten Optimum des physikalischen oder chemischen Prozesses kann unter Verwendung einer dritten prädiktiven Verteilung ermittelt werden. Die dritte prädiktive Verteilung kann eine dritte bedingte Wahrscheinlichkeitsverteilung des ersten statistischen Modells mit der Konditionierung auf die bekannten Messpunkte und auf die mehreren approximierten Maximalwerte des zweiten statistischen Modells sein. Die in diesem Absatz beschriebenen Merkmale in Kombination mit dem siebzehnten Beispiel bildet ein achtzehntes Beispiel.

Das Ermitteln eines approximierten Maximalwertes der mehreren approximierten Maximalwerte kann das Ermitteln eines dritten statistischen Modells unter Verwendung einer vierten prädiktiven Verteilung aufweisen, wobei die vierte prädiktive Verteilung eine vierte bedingte Wahrscheinlichkeitsverteilung des zweiten statistischen Modells mit der Konditionierung auf die bekannten Messpunkte sein kann, und wobei der approximierte Maximalwert ein Maximalwert des Ausgangsparameters des dritten statistischen Modells sein kann. Die in diesem Absatz beschriebenen Merkmale in Kombination dem achtzehnten Beispiel bildet ein neunzehntes Beispiel.

Die vierte prädiktive Verteilung kann mittels einer Sparse-Spectrum-Gauß-Prozess-Approximation (SSGP-Approximation) unter Verwendung einer fünften prädiktiven Verteilung ermittelt werden. Die fünfte prädiktive Verteilung kann eine bedingte Wahrscheinlichkeitsverteilung des ersten statistischen Modells ohne Rauschanteil mit der Konditionierung auf die bekannten Messpunkte sein. Die in diesem Absatz beschriebenen Merkmale in Kombination mit dem neunzehnten Beispiel bildet ein zwanzigstes Beispiel.

Das dritte statistische Modell kann unter Verwendung eines vierten statistischen Modells ermittelt werden, wobei das vierte statistische Modell unter Verwendung der fünften prädiktiven Verteilung ermittelt werden kann, wobei das vierte statistische Modell einen ersten Merkmalsvektor aufweisen kann, und wobei die fünfte prädiktive Verteilung als eine Superposition von ersten Merkmalsvektoren dargestellt werden kann. Die einzelnen Komponenten des ersten Merkmalsvektors können Cosinus-Funktionen mit "zufälliger" Frequenz und "zufälliger" Phasenverschiebung sein. Die Frequenzen, die Phasenverschiebungen und Wichtungen für die Superposition können derart ausgewählt werden, dass die fünfte Verteilung approximiert wird. Die in diesem Absatz beschriebenen Merkmale in Kombination mit dem neunzehnten Beispiel oder dem zwanzigsten Beispiel bilden ein einundzwanzigstes Beispiel.

Das dritte statistische Modell kann unter Verwendung eines zweiten Merkmalvektors ermittelt werden. Der zweite Merkmalsvektor kann sich von dem ersten Merkmalsvektor dahingehend unterscheiden, dass die Komponenten des zweiten Merkmalsvektors mit einer Amplitude multipliziert werden können, wobei die Größe der Amplitude von der Unsicherheit der Eingangsparameterwerte abhängen kann. Die in diesem Absatz beschriebenen Merkmale in Kombination mit einem dem einundzwanzigsten Beispiel bilden ein zweiundzwanzigstes Beispiel.

Die Entropie des durch die bekannten Messpunkte beschriebenen ersten statistischen Modells und mehreren approximierten Maximalwerten des Ausgangsparameters des zweiten statistischen Modells an dem robusten Optimum des physikalischen oder chemischen Prozesses kann mittels eines Verwerfungsverfahrens (Rejection Sampling) oder mittels Expectation Propagation ermittelt werden. Das Ermitteln der Entropie mittels Verwerfungsverfahren oder mittels Expectation Propagation hat den Vorteil, dass die erwartete Entropie, die analytisch nicht lösbar ist, approximiert werden kann. Expectation Propagation hat ferner den Vorteil, dass weniger Rechenaufwand benötigt wird. Die in diesem Absatz beschriebenen Merkmale in Kombination mit einem des siebzehnten Beispiels bis dem zweiundzwanzigsten Beispiel bildet ein dreiundzwanzigstes Beispiel.

Ein Computerprogrammprodukt kann Programminstruktionen speichern, welche, wenn sie ausgeführt werden, das Verfahren nach einem oder mehreren des ersten Beispiels bis dem dreiundzwanzigsten Beispiel ausführen. Das in diesem Absatz beschriebene Merkmal bildet ein fünfundzwanzigstes Beispiel.

Ein System kann eine Vorrichtung nach dem vierundzwanzigsten Beispiel aufweisen. Das System kann ferner zumindest einen Sensor aufweisen, der eingerichtet sein kann, der Vorrichtung Eingangsparameterwerten zugeordnete Ausgangsparameterwerte bereitzustelle. Das System mit den in diesem Absatz beschriebenen Merkmalen bildet ein sechsundzwanzigstes Beispiel.

Ein Produktionssystem kann eine Vorrichtung nach dem vierundzwanzigsten Beispiel aufweisen. Das Produktionssystem kann ferner eine Produktionsvorrichtung aufweisen, die eingerichtet sein kann, ein Produkt herzustellen. Das Produktionssystem kann zumindest einen Sensor aufweisen, der eingerichtet sein kann, der Vorrichtung Eingangsparameterwerten zugeordnete Ausgangsparameterwerte bereitzustellen. Das Produktionssystem kann eine Steuervorrichtung aufweisen, die eingerichtet sein kann, die Produktionsvorrichtung derart zu steuern, dass ein Eingangsparameterwert in Abhängigkeit des Eingangsparameterwertes des robusten Optimums gewählt wird, insbesondere diesem entspricht. Das Produktionssystem mit den in diesem Absatz beschriebenen Merkmalen bildet ein siebenundzwanzigstes Beispiel.

Ein Bearbeitungssystem kann eine Vorrichtung nach dem vierundzwanzigsten Beispiel aufweisen. Das Bearbeitungssystem kann ferner eine Bearbeitungsvorrichtung aufweisen, die eingerichtet sein kann, ein Werkstück zu bearbeiten. Das Bearbeitungssystem kann zumindest einen Sensor aufweisen, der eingerichtet sein kann, der Vorrichtung Eingangsparameterwerten zugeordnete Ausgangsparameterwerte bereitzustellen. Das Bearbeitungssystem kann eine Steuervorrichtung aufweisen, die eingerichtet sein kann, die Bearbeitungsvorrichtung derart zu steuern, dass ein Eingangsparameterwert in Abhängigkeit des Eingangsparameterwertes des robusten Optimums gewählt wird, insbesondere diesem entspricht. Das Bearbeitungssystem kann ein chemisches Bearbeitungssystem oder ein mechanisches Bearbeitungssystem sein. Das Bearbeitungssystem mit den in diesem Absatz beschriebenen Merkmalen bildet ein achtundzwanzigstes Beispiel.

Ein Robotersystem kann eine Vorrichtung nach dem vierundzwanzigsten Beispiel aufweisen. Das Robotersystem kann ferner eine Robotervorrichtung aufweisen, die eingerichtet sein kann, eine Bewegung auszuführen. Das Robotersystem kann zumindest einen Sensor aufweisen, der eingerichtet sein kann, der Vorrichtung Eingangsparameterwerten zugeordnete Ausgangsparameterwerte bereitzustellen. Das Robotersystem kann eine Steuervorrichtung aufweisen, die eingerichtet sein kann, die Robotervorrichtung derart zu steuern, dass ein Eingangsparameterwert in Abhängigkeit des Eingangsparameterwertes des robusten Optimums gewählt wird, insbesondere diesem entspricht. Das Robotersystem mit den in diesem Absatz beschriebenen Merkmalen bildet ein neunundzwanzigstes Beispiel.

Ausführungsbeispiele der Erfindung sind in der Zeichnung dargestellt und in der nachfolgenden Beschreibung näher erläutert.

Es zeigen

Figur 1A    eine Vorrichtung gemäß verschiedenen Ausführungsformen;

Figur 1B    eine Vorrichtung gemäß verschiedenen Ausführungsformen;

Figur 2    ein Verarbeitungssystem zum Auswählen eines neuen Messpunktes gemäß verschiedenen Ausführungsformen;

Figur 3    ein Verarbeitungssystem zum Auswählen eines neuen Messpunktes gemäß verschiedenen Ausführungsformen;

Figur 4    beispielhafte Verteilung eines ersten statistischen Modells;

Figur 5    beispielhafte Verteilung eines zweiten statistischen Modells;

Figur 6    ein Verarbeitungssystem zum Adaptieren eines statistischen Modells gemäß verschiedenen Ausführungsformen;

Figur 7    ein Verarbeitungssystem zum Ermitteln eines robusten Optimums eines physikalischen oder chemischen Prozesses gemäß verschiedenen Ausführungsformen;

Figur 8A    ein Verfahren zum Auswählen eines neuen Messpunktes eines physikalischen oder chemischen Prozesses gemäß verschiedenen Ausführungsformen;

Figur 8B    ein Verfahren zum Ermitteln eines robusten Optimums eines physikalischen oder chemischen Prozesses gemäß verschiedenen Ausführungsformen;

Figur 9A    ein System zum Ermitteln eines robusten Optimums eines physikalischen oder chemischen Prozesses gemäß verschiedenen Ausführungsformen;

Figur 9B    ein System, das ein gemäß verschiedenen Ausführungsformen adaptiertes statistisches Modell verwendet.

In einer Ausführungsform kann eine "Schaltung" als jede Art von Logik-implementierender Entität verstanden werden, welche Hardware, Software, Firmware oder eine Kombination davon sein kann. Daher kann in einer Ausführungsform eine "Schaltung" eine hartverdrahtete Logikschaltung oder eine programmierbare Logikschaltung, wie beispielsweise ein programmierbarer Prozessor, zum Beispiel ein Mikroprozessor (z.B. ein CISC (Prozessor mit großem Befehlsvorrat) oder ein RISC (Prozessor mit reduziertem Befehlsvorrat)), sein. Eine "Schaltung" kann auch Software sein, die von einem Prozessor implementiert bzw. ausgeführt wird, zum Beispiel jede Art von Computerprogramm, zum Beispiel ein Computerprogramm das einen virtuellen Maschinencode, wie beispielsweise Java, verwendet. Jede andere Art der Implementierung der jeweiligen Funktionen, die im Folgenden ausführlicher beschrieben werden, kann in Übereinstimmung mit einer alternativen Ausführungsform als eine "Schaltung" verstanden werden.

Verschiedene Ausführungsbeispiele betreffen eine Vorrichtung und ein Verfahren zum Ermitteln eines robusten Optimums eines physikalischen oder chemischen Prozesses gemäß einem Bayes-Optimierungsverfahren. Die Prozessparameter bei physikalischen oder chemischen Prozessen können aufgrund von Störungen bzw. Rauschen von den eingestellten Werten abweichen, wodurch sich gewünschte Eigenschaften zum Beispiel eines Werkstücks verschlechtern können bzw. vorgegebene Eigenschaften nicht erfüllt werden können. Anschaulich wird ein statistisches Modell bereitgestellt, das imstande ist, ein robustes Optimum des physikalischen oder chemischen Prozesses zu ermitteln, das robust gegenüber Veränderungen der Prozessparameter ist, d.h. dass die gewünschten bzw. vorgegebenen Eigenschaften trotz Störungen bzw. Rauschen der Prozessparameter vorliegen.

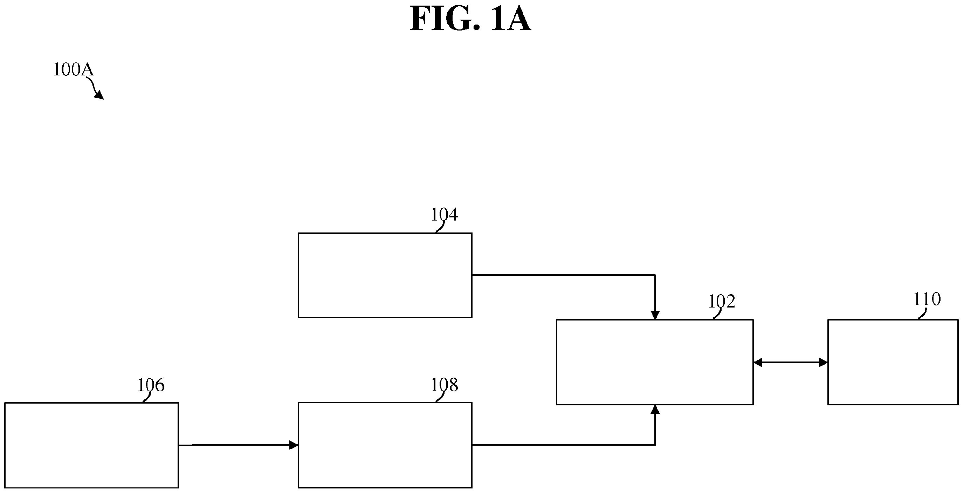

**FIG. 1A** stellt eine Vorrichtung 100A gemäß verschiedenen Ausführungsformen dar. Die Vorrichtung 100A kann eine Speichervorrichtung 102 aufweisen. Die Speichervorrichtung 102 kann zumindest einen Speicher aufweisen. Der Speicher kann beispielsweise bei der durch einen Prozessor durchgeführten Verarbeitung verwendet werden. Ein in den Ausführungsformen verwendeter Speicher kann ein flüchtiger Speicher, zum Beispiel ein DRAM (dynamischer Direktzugriffsspeicher), oder ein nichtflüchtiger Speicher, zum Beispiel ein PROM (programmierbarer Festwertspeicher), ein EPROM (löschbarer PROM), ein EEPROM (elektrisch löschbarer PROM) oder ein Flash-Speicher, wie beispielsweise eine Speichereinrichtung mit schwebendem Gate, eine ladungsabfangende Speichereinrichtung, ein MRAM (magnetoresistiver Direktzugriffsspeicher) oder ein PCRAM (Phasenwechsel-Direktzugriffsspeicher), sein. Die Speichervorrichtung 102 kann eingerichtet sein, um bereitgestellte Parameterwerte zu speichern. Die Speichervorrichtung 102 kann eingerichtet sein, um bereitgestellte mehrere Eingangsparameterwerte 104 eines physikalischen oder chemischen Prozesses zu speichern. Die Vorrichtung 100A kann mindestens einen Sensor 106 aufweisen, wobei der Sensor 106 eingerichtet sein kann, um mehrere Ausgangsparameterwerte 108 des physikalischen oder chemischen Prozesses bereitzustellen. Jeder Ausgangsparameterwert der mehreren Ausgangsparameterwerte 108 kann genau einem Eingangsparameterwert der mehreren Eingangsparameterwerte 104 zugeordnet sein, wobei ein Eingangsparameterwert und der dem Eingangsparameterwert zugeordnete gemessene Ausgangsparameterwert einen Messpunkt bilden können. Der einem Eingangsparameterwert zugeordnete Eingangsparameter kann eine physikalische Größe sein. Der Eingangsparameter kann ein prozessbezogener bzw. prozessrelevanter Parameter sein, wie beispielsweise eine Prozesstemperatur, eine Prozesszeit, ein Vakuum bzw. eine Gasatmosphäre usw. Der Eingangsparameter kann prozessrelevante Eigenschaften von Materialien sein, wie beispielsweise Härte, Wärmeleitfähigkeit, elektrische Leitfähigkeit, Dichte, Mikrostruktur, Makrostruktur, chemische Zusammensetzung usw. Der einem Ausgangsparameterwert zugeordnete Ausgangsparameter kann eine physikalische Größe sein. Der Ausgangsparameter kann ein anwendungsspezifisches Qualitätskriterium aufweisen. Der Ausgangsparameter kann ein bauteilbezogener Parameter sein, wie beispielsweise ein Maß oder eine Schichtdicke, oder kann ein materialbezogener Parameter sein, wie beispielsweise Härte, Wärmeleitfähigkeit, elektrische Leitfähigkeit, Dichte, chemische Zusammensetzung usw. Es kann ein gewünschter Wert für den Ausgangsparameter vordefiniert sein. Das heißt, es kann zum Beispiel eine gewünschte Härte oder eine gewünschte elektrische Leitfähigkeit vordefiniert sein.

Die Vorrichtung 100A kann ferner mindestens einen Prozessor 110 aufweisen. Der Prozessor 110 kann, wie oben beschrieben, jede Art von Schaltung, d.h. jede Art von Logik-implementierender Entität, sein. Der Prozessor 110 kann ein Grafikprozessor (GPU) sein und der Grafikprozessor kann einen zugeordneten Grafikspeicher (Video-RAM) bei der Datenverarbeitung verwenden. In verschiedenen Ausführungsformen ist der Prozessor 110 eingerichtet, die mehreren Eingangsparameterwerte 104 und die mehreren Ausgangsparameterwerte 108 zu verarbeiten.

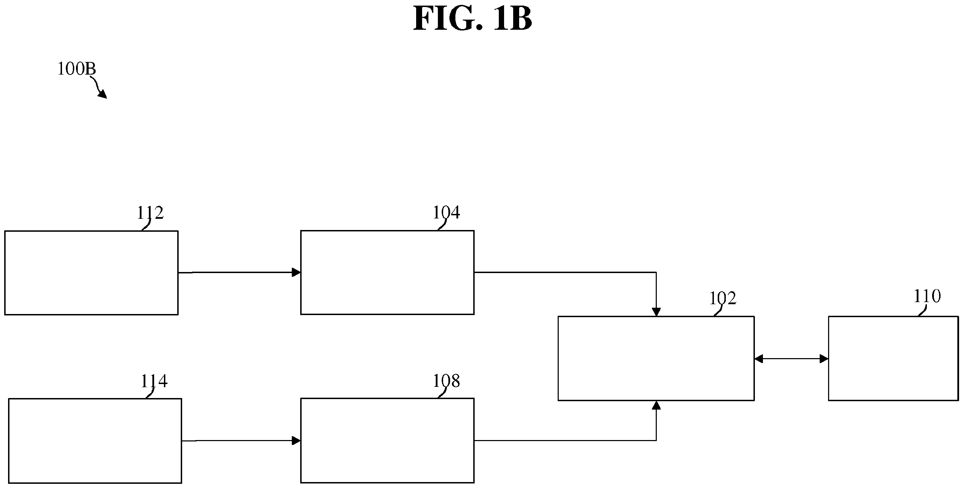

FIG. 1B stellt eine Vorrichtung 100B gemäß verschiedenen Ausführungsformen dar. Die Vorrichtung 100B kann im Wesentlichen der Vorrichtung 100A entsprechen, wobei die Vorrichtung 100B einen ersten Sensor 112 und einen zweiten Sensor 114 aufweist und wobei der erste Sensor 112 eingerichtet sein kann, der Speichervorrichtung 102 die mehreren Eingangsparameterwerte 104 bereitzustellen, und wobei der zweite Sensor 114 eingerichtet sein kann, der Speichervorrichtung die mehreren Ausgangsparameterwerte 108 bereitzustellen.

Gemäß verschiedenen Ausführungsformen kann die Vorrichtung 100A und/oder die Vorrichtung 100B ferner zusätzliche Sensoren aufweisen, wobei jeder zusätzliche Sensor der zusätzlichen Sensoren eingerichtet sein kann, der Speichervorrichtung 102 Parameterwerte bereitzustellen, die einem jeweiligen Eingangsparameterwert der mehreren Eingangsparameterwerte 104 zugeordnet sind.

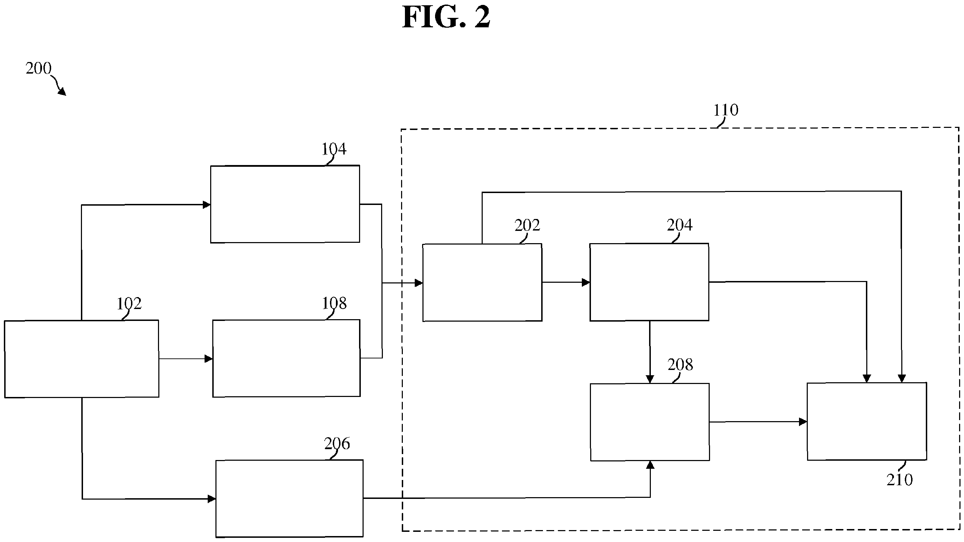

FIG. 2 stellt Verarbeitungssystem 200 zum Auswählen eines neuen Messpunktes gemäß verschiedenen Ausführungsformen dar. Das Verarbeitungssystem 200 kann die Speichervorrichtung 102 zum Speichern der mehreren Eingangsparameterwerte 104 und der mehreren Ausgangsparameterwerte 108 aufweisen. Das Verarbeitungssystem 200 kann ferner den mindestens einen Prozessor 110 aufweisen. Der Prozessor 110 kann eingerichtet sein, die mehreren Eingangsparameterwerte 104 und die mehreren Ausgangsparameterwerte 108 zu verarbeiten. Der Prozessor 110 kann eingerichtet sein, um bekannte Messpunkte 202 zu ermitteln, wobei jeder bekannte Messpunkt der bekannten Messpunkte 202 einen Eingangsparameterwert der mehreren Eingangsparameterwerte 104 und einen dem Eingangsparameterwert zugeordneten Ausgangsparameterwert der mehreren Ausgangsparameterwerte 108 aufweist.

Die bekannten Messpunkte 202 ($D_n$) können durch Gleichung (1) beschrieben werden:

$$D_n = \{(x_i, y_i)\}_{i=1:n} \qquad (1)$$

wobei $x_i$ ein Eingangsparameterwert der mehreren Eingangsparameterwerte 104 und $y_i$ der dem Eingangsparameterwert zugeordnete Ausgangsparameterwert ist und wobei n die Anzahl an bekannten Messpunkten 202 ist. Es ist anzumerken, dass die bekannten Messpunkte 202 ferner zusätzliche Parameterwerte aufweisen können, die einem jeweiligen Eingangsparameterwert der mehreren Eingangsparameterwerte 104 zugeordnet sind.

Der Prozessor 110 kann eingerichtet sein, ein erstes statistisches Modell 204 zu ermitteln.

Das erste statistische Modell 204 beschreibt einen physikalischen oder chemischen Prozess zu den bekannten Messpunkten 202. Das erste statistische Modell 204 kann eine erste Funktion sein, die den physikalischen oder chemischen Prozess zu den bekannten Messpunkten 202 beschreibt. Anders ausgedrückt, kann das erste statistische Modell 204 für einen Eingangsparameterwert einen zugeordneten Ausgangsparameterwert ausgeben.

Noch anders ausgedrückt, beschreibt das erste statistische Modell 204 den Ausgangsparameter als Funktion des Eingangsparameters. Das heißt, das erste statistische Modell 204 beschreibt einen Zusammenhang der Eingangsparameterwerte und Ausgangsparameterwerte eines physikalischen oder chemischen Prozesses. Das erste statistische Modell 204 kann durch die Funktion *f(x)* beschrieben werden. Ein gemessener Ausgangsparameterwert kann einen Rauschanteil aufweisen. Das heißt, ein gemessener Ausgangsparameterwert kann von der Funktion des ersten statistischen Modells 204 abweichen. Der Prozessor 110 kann eingerichtet sein, ein erstes statistisches Modell zu ermitteln, das ferner einen Rauschanteil $\epsilon$ berücksichtigt und durch *y(x)* nach Gleichung (2) beschrieben werden kann:

$$y(x) = f(x) + \epsilon \qquad (2)$$

wobei der Rauschanteil $\epsilon$ durch eine Normalverteilung beschrieben werden kann als $\epsilon \sim \mathcal{N}(0, \sigma_\epsilon^2)$ . Im Folgenden wird das erste statistische Model 204 durch *y(x)* beschrieben und das erste statistische Model 204 ohne Rauschanteil durch *f(x)* beschrieben.

Das erste statistische Modell 204 ohne Rauschanteil kann mittels einer Gauß-Prozess-Regression (GP-Regression) unter Verwendung der bekannten Messpunkte 202 ermittelt, zum Beispiel approximiert, werden. Das erste statistische Modell 204 ohne Rauschanteil kann von einem bayesschen neuronalen Netzwerk gebildet werden. Das erste statistische Modell 204 ohne Rauschanteil kann durch ein anderes Modell ermittelt werden, das Konfidenzintervalle ausgibt. Im Folgenden wird auf das Ermitteln des ersten statistischen Modells 204 ohne Rauschanteil durch eine GP-Regression Bezug genommen. Das erste statistische Modell 204 ohne Rauschanteil kann mehrere Datenpunkte $X$ in dem Zahlenbereich der reellen Zahlen beschreiben, d.h. $f(x): \mathcal{X} \mapsto \mathbb{R}$ mit $\mathcal{X} \subseteq \mathbb{R}^d$ , wobei *d* die Dimension ist. Die Dimension wird durch die Anzahl an den einem jeweiligen Eingangsparameterwert zugeordneten Parameterwerten bestimmt. Das heißt, dass für den Fall, dass den mehreren Eingangsparameterwerten 104 mehrere Ausgangsparameterwerte 108 zugeordnet sind, die Dimension *d*=2 ist.

Die GP-Regression definiert eine vorausgehende Verteilung mit einem Mittelwert gleich "0" und einer Kovarianz, die durch eine Kernfunktion $k_f(x, x')$ für alle $x, x' \in X$, gegeben sein kann.

Durch eine Konditionierung der vorausgehenden Verteilung auf die bekannten Messpunkte 202 kann ein prädiktiver Mittelwert $m_f$ mittels Gleichung (3) und eine Varianz $v_f$ mittels Gleichung (4) ermittelt werden:

$$m_f(x|D_n) = k_f(x)^\top K^{-1} y \qquad (3)$$

$$v_f(x|D_n) = k_f(x,x) - k_f(x)^\top K^{-1} k_f(x) \qquad (4)$$

wobei $[k_f(x)]_i = k_f(x, x_i)$; $[K]_{ij} = k_f(x_i, x_j) + \delta_{ij}\sigma_\epsilon^2$ ; $[y]_i = y_i$ und wobei $\delta_{ij}$ das Kronecker-Delta ist.

Die Speichervorrichtung 102 kann ferner eine Veränderung der Eingangsparameterwerte 206 speichern. Die Veränderung der Eingangsparameterwerte 206 kann vordefiniert sein. Die Veränderung der Eingangsparameterwerte 206 kann eine gewünschte oder erforderliche maximale Abweichung eines Eingangsparameterwertes angeben. Die Veränderung der Eingangsparameterwerte 206 kann eine erforderlich oder erwünschte Größe eines Prozessfensters definieren. Beispielsweise kann der Eingangsparameter eine Prozesstemperatur angeben und die Veränderung der Eingangsparameterwerte 206 von einer eingestellten Prozesstemperatur kann zum Beispiel 5 K oder zum Beispiel 10 % sein. Gemäß verschiedenen Ausführungsformen ist die Veränderung der Eingangsparameterwerte 206 durch eine Verteilung (zum Beispiel eine Normalverteilung) gegeben. Gemäß verschiedenen Ausführungsformen ist der Prozessor 110 eingerichtet, die Veränderung der Eingangsparameterwerte 206 unter Verwendung der mehreren Eingangsparameterwerte 104 und der mehreren Ausgangsparameterwerte 108 zu ermitteln.

Der Prozessor 110 ist ferner eingerichtet, ein zweites statistisches Modell 208 zu ermitteln. Das zweite statistische Modell 208 kann unter Verwendung des ersten statistisches Modell 204 und der Veränderung der Eingangsparameterwerte 206 ermittelt werden.

Das zweite statistisches Modell 208 (*g(x)*) kann mittels Gleichung (5) ermittelt werden:

$$g(x) = \mathbb{E}_{\xi \sim p(\xi)}[f(x + \xi)] \qquad (5)$$

wobei $\xi$ die Veränderung der Eingangsparameterwerte 206 ist. Das hat den Vorteil, dass das zweite statistische Modell 208 eine Robustheit für die Ausgangsparameterwerte des physikalischen oder chemischen Prozesses bezüglich einer Veränderung der Eingangsparameterwerte beschreibt. Das zweite statistische Modell 208 kann durch eine mathematische Faltung mittels Gleichung (6) ermittelt werden:

$$g(x) = \mathbb{E}_{\xi \sim p(\xi)}[f(x + \xi)] = \int f(x + \xi)p(\xi)d\xi \qquad (6)$$

wobei $p(\xi)$ eine Normalverteilung mit $p(\xi) \sim \mathcal{N}(0, \Sigma_x)$ ist, und

wobei $\Sigma_x = diag[\sigma_{x,1}^2, ..., \sigma_{x,d}^2]$ ist. Das heißt, die Verteilung der Veränderung der Eingangsparameterwerte 206 kann unter Verwendung der Varianz der Eingangsparameterwerte eines jeweiligen Messpunktes der bekannten Messpunkte 202 ermittelt werden.

Wird, wie oben beschrieben, das erste statistische Modell 204 mittels einer GP-Regression ermittelt, so kann ein prädiktiver Mittelwert $m_g$ des zweiten statistischen Modells 208 mittels Gleichung (7) und eine Varianz $v_g$ mittels Gleichung (8) ermittelt werden:

$$m_g(x|D_n) = k_{gf}(x)^\top K^{-1}y \qquad (7)$$

$$v_g(x|D_n) = k_{gf}(x, x) - k_{gf}(x)^\top K^{-1}k_{fg}(x) \qquad (8)$$

wobei $k_g(x, x') = \iint k_f(x + \xi, x' + \xi')p(\xi)p(\xi')d\xi d\xi'$, und
wobei $k_{gf}(x, x') = \int k_f(x + \xi, x')p(\xi)d\xi$ ist.

Der Prozessor 110 ist ferner dazu eingerichtet, einen neuen Messpunkt 210 des Eingangsparameters auszuwählen. Der neue Messpunkt 210 kann Eingangsparameterwert sein, für den durch Ermitteln des zugehörigen Ausgangsparameterwertes ein robustes Optimum des ersten statistischen Modells 204 ermittelt werden kann.

Ein einem Eingangsparameterwert im Bereich eines robusten Optimums zugeordneter Ausgangsparameterwert wird durch eine Abweichung, wie beispielsweise der Veränderung der Eingangsparameterwerte 206, von dem Eingangsparameterwert des robusten Optimums gar nicht oder nur geringfügig kleiner. Das heißt, die Ausgangsparameterwerte sind im Bereich des robusten Optimums robust bezüglich einer Veränderung der Eingangsparametwerte. Anders ausgedrückt, kann bei einer gleichen Verteilung (zum Beispiel einer Gauß-Verteilung oder einer Gleichverteilung) der Peaks des robusten Optimums 404 und des globalen Maximums 402 des ersten statistischen Modells 204 das robuste Optimum 404 eine größere Halbwertsbreite als das globale Maximum 402 aufweisen. Das robuste Optimum 404 kann in Abhängigkeit der Veränderung der Eingangsparameterwerte 206 ermittelt werden. Das heißt, das erste statistische Modell 204 kann mehrere robuste Optima aufweisen, wobei jedes robuste Optimum der mehreren robusten Optima ein robustes Optimum für eine Veränderung der Eingangsparameterwerte 206 bzw. für einen Bereich an Veränderungen der ersten Parametwerte ist.

Der neue Messpunkt 210 wird unter Verwendung des ersten statistischen Modells 204 und des zweiten statistischen Modells 208 mit Konditionierung auf einen angenommenen Maximalwert des Ausgangsparameters des zweiten statistischen Modells 208 ermittelt. Der angenommene Maximalwert des Ausgangsparameters des zweiten statistischen Modells 208 kann der einem globalen Maximum des zweiten statistischen Modells 208 zugeordnete Ausgangsparameterwert sein. Das heißt, der Maximalwert ($g^*$) kann durch $g^* = max_{x \in X}g(x)$ ermittelt werden. Der neue Messpunkt 210 wird derart ausgewählt, dass eine Differenz aus der ersten Entropie 304 und der erwarteten Entropie 306 an dem neuen Messpunkt 210 im Wesentlichen maximal ist oder in einem vorgegebenen Umgebungsbereich des Maximums liegt.

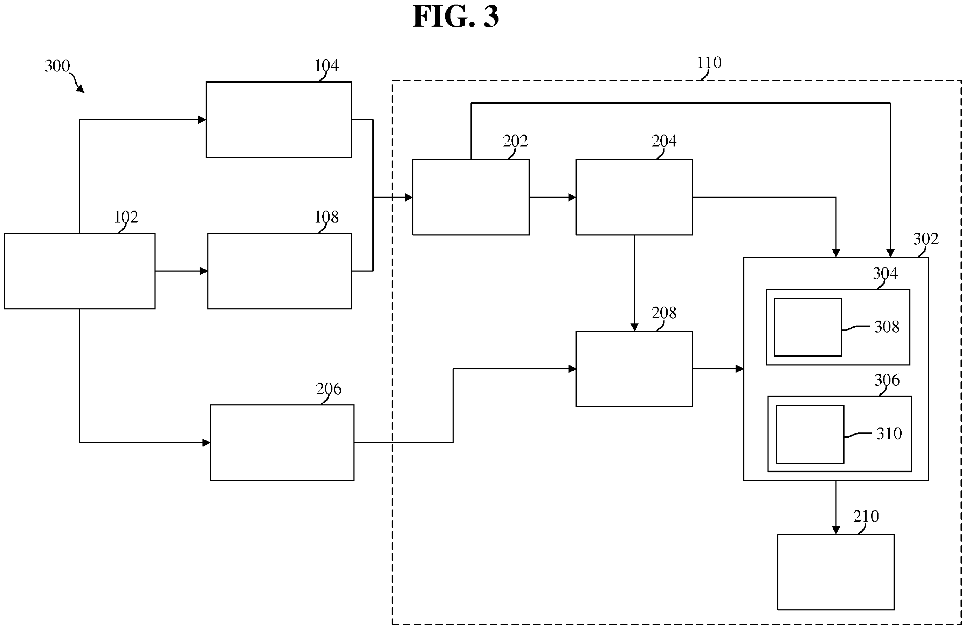

**FIG. 3** stellt Verarbeitungssystem 300 zum Auswählen eines neuen Messpunktes gemäß verschiedenen Ausführungsformen dar. Das Verarbeitungssystem 300 kann im Wesentlichen dem Verarbeitungssystem 200 entsprechen, wobei der neue Messpunkt 210 durch eine Akquisitionsfunktion 302 ermittelt wird. Der neue Messpunkt 210 kann der einem globalen Maximum der Akquisitionsfunktion 302 zugeordnete Eingangsparameterwert sein. Die Akquisitionsfunktion 302 kann eine Transinformation (auch gegenseitige Information genannt) des ersten statistischen Modells 204 und des Maximalwertes des zweiten statistischen Modells 208 sein. Die Akquisitionsfunktion 302 kann eine bedingte Transinformation des ersten statistischen Modells 204 und des Maximalwertes des zweiten statistischen Modells 208 mit der Konditionierung auf die bekannten Messpunkte 202 sein.

Die Akquisitionsfunktion 302 ($\alpha_{NES}(x)$) kann durch Gleichung (9) beschrieben werden:

$$\alpha_{NES}(x) = I\big((x, y); g^*|D_n\big) \qquad (9)$$

Die bedingte Transinformation des ersten statistischen Modells 204 und des Maximalwertes des zweiten statistischen Modells 208 mit der Konditionierung auf die bekannten Messpunkte 202 kann unter Verwendung einer ersten Entropie 304, beispielsweise einer ersten differentiellen Entropie, und einer erwarteten Entropie 306, beispielsweise einer erwarteten differentiellen Entropie ermittelt werden. Die erste Entropie 304 kann eine Entropie des durch die bekannten Messpunkte 202 beschriebenen ersten statistischen Modells 204 für den physikalischen oder chemischen Prozess an einem Eingangsparameterwert sein. Die erwarte Entropie 306 kann eine erwartete Entropie des durch die bekannten Messpunkte 202 beschriebenen ersten statistischen Modells 204 an einem Eingangsparameterwert und dem angenommenen Maximalwert des Ausgangsparameters des zweiten statistischen Modells 208 an dem robusten Optimum des physikalischen oder chemischen Prozesses sein.

Die erste Entropie 304 kann unter Verwendung einer ersten prädiktiven Verteilung 308 ermittelt werden. Die erste prädiktive Verteilung 308 kann eine erste bedingte Wahrscheinlichkeitsverteilung des ersten statistischen Modells 204 mit der Konditionierung auf die bekannten Messpunkte 202 sein. Die erwartete Entropie 306 kann unter Verwendung einer zweiten prädiktiven Verteilung 310 ermittelt werden. Die zweite prädiktive Verteilung 310 kann eine zweite bedingte Wahrscheinlichkeitsverteilung des ersten statistischen Modells 204 mit der Konditionierung auf die bekannten Messpunkte 202 und auf den angenommenen Maximalwert des Ausgangsparameters des zweiten statistischen Modells 208 an dem robusten Optimum sein.

Die Akquisitionsfunktion 302 ($\alpha_{NES}(x)$) stellt. eine Differenz aus der ersten Entropie 304 und der erwarteten Entropie 306 dar srnr und kann durch Gleichung (10) beschrieben werden:

$$\alpha_{NES}(x) = H[p(y(x)|D_n)] - \mathbb{E}_{g^*|D_n}\big[H[p(y(x)|D_n, g^*)]\big] \qquad (10)$$

wobei $H[p(y(x)|D_n)]$ die erste Entropie 304 mit der ersten prädiktiven Verteilung 308 $p(y(x)|D_n)$ ist und wobei $\mathbb{E}_{g^*|D_n}H[p(y(x)|D_n, g^*)]$ die erwartete Entropie 306 mit der zweiten prädiktiven Verteilung 310 $p(y(x)|D_n,g^*)$ ist.

Die erste prädiktive Verteilung 308 kann eine Gauß-Verteilung sein. Die erste prädiktive Verteilung 308 kann durch einen Gauß-Prozess (GP) ermittelt werden. Die erste Entropie 304 kann mittels Gleichung (11) ermittelt, beispielsweise berechnet, werden:

$$H[p(y(x)|D_n)] = 0.5 \log[2\pi e(v_f(x|D_n) + \sigma_\epsilon^2)] \qquad (11)$$

Die erwartete Entropie 306 kann durch eine Monte-Carlo-Approximation ermittelt, beispielsweise approximiert, werden. Die erwartete Entropie 306 kann unter Verwendung einer zweiten Entropie, beispielsweise einer zweiten differentiellen Entropie, ermittelt werden. Die zweite Entropie kann eine Entropie des durch die bekannten Messpunkte 202 beschriebenen ersten statistischen Modells 204 und mehreren approximierten Maximalwerten des Ausgangsparameters des zweiten statistischen Modells 208 an dem robusten Optimum des physikalischen oder chemischen Prozesses sein. Die zweite Entropie kann unter Verwendung einer dritten prädiktiven Verteilung ermittelt werden. Die dritte prädiktive Verteilung kann eine dritte bedingte Wahrscheinlichkeitsverteilung des ersten statistischen Modells 204 mit der Konditionierung auf die bekannten Messpunkte 202 und auf mehrere approximierte Maximalwerte des Ausgangsparameters des zweiten statistischen Modells 208 sein.

Die erwartete Entropie 306 kann mittels Gleichung (12) ermittelt, beispielsweise approximiert, werden:

$$\mathbb{E}_{g^*|D_n}\big[H[p(y(x)|D_n, g^*)]\big] \approx \frac{1}{K}\sum_{g_k^*\in G^*} H[p(y(x)|D_n, g_k^*)] \qquad (12)$$

wobei $H[p(y(x)|D_n, g_k^*)]$ die zweite Entropie mit der dritten prädiktiven Verteilung $p(y(x)|D_n, g_k^*)$ ist, wobei $G^*$ ein Satz mit der Anzahl von $K$ Stichproben von $p(g^*|D_n)$ ist, und wobei $g_k^*$ ein approximierter Maximalwert der mehreren approximierten Maximalwerte des zweiten statistischen Modells 208 ist.

Ein approximierter Maximalwert ($g_k^*$) der mehreren approximierten Maximalwerte des Ausgangsparameters kann unter Verwendung eines dritten statistischen Modells ($\hat{g}_k(x)$) ermittelt werden. Ein approximierter Maximalwert der mehreren approximierten Maximalwerte kann ein Maximalwert des dritten statistischen Modells sein, d.h. $g_k^* = max_{x\in\mathcal{X}}\hat{g}_k(x)$. Das dritte statistische Modell kann unter Verwendung einer vierten prädiktiven Verteilung ermittelt werden. Die vierte prädiktive Verteilung kann eine vierte bedingte Wahrscheinlichkeitsverteilung des zweiten statistischen Modells 208 mit der Konditionierung auf die bekannten Messpunkte 202 sein, d.h. die vierte prädiktive Verteilung kann durch $p(g(x)|D_n)$ beschrieben werden. Die vierte prädiktive Verteilung kann mittels einer Sparse-Spectrum-Gauß-Prozess-Approximation (SSGP-Approximation) ermittelt werden. Das dritte statistische Modell kann unter Verwendung eines vierten statistischen Modells ermittelt werden. Das vierte statistische Modell kann unter Verwendung einer fünften prädiktiven Verteilung ermittelt werden. Die fünfte prädiktive Verteilung kann eine bedingte Wahrscheinlichkeitsverteilung des ersten statistischen Modells 204 ohne Rauschanteil mit der Konditionierung auf die bekannten Messpunkte 202 sein, d.h. die fünfte prädiktive Verteilung kann durch $p(f(x)|D_n)$ beschrieben werden. Das vierte statistische Modell kann einen ersten Merkmalsvektor aufweisen, wobei die fünfte prädiktive Verteilung als eine Superposition von ersten Merkmalsvektoren dargestellt werden kann. Die einzelnen Komponenten des ersten Merkmalsvektors können Cosinus-Funktionen mit "zufälliger" Frequenz und "zufälliger" Phasenverschiebung sein. Die Frequenzen, die Phasenverschiebungen und Wichtungen für die Superposition können derart ausgewählt werden, dass die fünfte Verteilung approximiert wird.

Die vierte prädiktive Verteilung kann mittels einer SSGP-Approximation unter Verwendung der ersten prädiktiven Verteilung ermittelt werden.

Das vierte statistische Modell $\hat{f}_k(x)$ kann mittels Gleichung (13) ermittelt werden:

$$\hat{f}_k(x) = a^T \varphi_f(x) \qquad (13)$$

wobei $\varphi_f(x)$ der erste Merkmalsvektor mit $\varphi_f(x) \in \mathbb{R}^M$ ist und wobei $a$ ein Gewichtungsfaktor ist und nach $a \sim \mathcal{N}(A^{-1}\Phi_f^T y, \sigma_\epsilon^2 A^{-1})$ verteilt ist, wobei $A = \Phi_f^T \Phi_f + \sigma_\epsilon^2 I$ und $\Phi_f^T = [\varphi_f(x_1), \dots, \varphi_f(x_n)]$ ist.

Der erste Merkmalsvektor kann durch $\varphi_{f,i}(x) = \cos(w_i^T x + b_i)$ ermittelt werden, wobei $b_i \sim \mathcal{U}(0,2\pi)$ und $w_i \sim p(w) \propto s(w)$ ist.

Das dritte statistische Modell kann unter Verwendung des vierten statistischen Modells ermittelt werden, wobei das dritte statistische Modell $\hat{g}_k(x)$ mittels Gleichung (14) ermittelt werden kann:

$$\hat{g}_k(x) = a^T \varphi_g(x) \qquad (14)$$

wobei $\varphi_g(x)$ ein zweiter Merkmalsvektor ist. Der zweite Merkmalsvektor kann durch $\varphi_{g,i}(x) = \int \varphi_{g,i}(x+\xi)p(\xi)d\xi = \varphi_{f,i}(x) \exp\left(-\frac{1}{2}\sum_{j=1}^d w_{i,j}^2 \sigma_{x,j}^2\right)$ ermittelt werden. Der zweite Merkmalsvektor kann sich von dem ersten Merkmalsvektor dahingehend unterscheiden, dass die Komponenten des zweiten Merkmalsvektors mit einer Amplitude multipliziert werden können, wobei die Größe der Amplitude von der Unsicherheit der Eingangsparameterwerte abhängen kann.

Die zweite Entropie kann mittels Verwerfungsverfahren (Rejection Sampling) oder mittels Expectation Propagation ermittelt, beispielsweise approximiert, werden. Anders ausgedrückt, können die der zweiten Entropie zugeordneten mehreren approximierten Maximalwerte des Ausgangsparameters des zweiten statistischen Modell 208 mittels Verwerfungsverfahren (Rejection Sampling) oder mittels Expectation Propagation ermittelt, beispielsweise approximiert, werden.

Bei dem Verwerfungsverfahren kann das vierte statistische Modell $\hat{f}_k(x)$ unter Verwendung der ersten prädiktiven Verteilung 308 ($p(y(x)|D_n)$) ermittelt werden und das dritte statistische Modell kann unter Verwendung des vierten statistischen Modells ermittelt werden. Bei dem Verwerfungsverfahren kann ein approximierter Maximalwert ($g_k^*$) der mehreren approximierten Maximalwerte ausgewählt werden, wenn das Maximum des dritten statistischen Modells kleiner als der approximierte Maximalwert ist bzw. ein approximierter Maximalwert ($g_k^*$) der mehreren approximierten Maximalwerte kann verworfen werden, wenn das Maximum des dritten statistischen Modells größer als der approximierte Maximalwert ist.

Für die ausgewählten approximierten Maximalwerte kann die zweite Entropie mittels Gleichung (15) ermittelt, beispielsweise approximiert, werden:

$$H[p(y(x)|D_n, g_k^*)] \approx \tfrac{1}{L} \Sigma_{\hat{y} \in \hat{Y}} \ln[\hat{p}(\hat{y}_i)] \tag{15}$$

wobei $\hat{Y}$ ein Satz mit der Anzahl von $L$ ausgewählten approximierten Maximalwerten ist, und wobei $\hat{p}(\cdot)$ die Kerndichteschätzung ist.

Bei der Expectation Propagation kann die zweite Entropie unter Verwendung einer sechsten prädiktiven Verteilung ermittelt werden. Die sechste prädiktive Verteilung kann eine bedingte Wahrscheinlichkeitsverteilung des ersten statistischen Modells 204 ohne Rauschanteil mit der Konditionierung auf die bekannten Messpunkte 202 und auf die mehreren approximierten Maximalwerte sein, d.h. die sechste prädiktive Verteilung kann durch $p(f(x)|D_n, g_k^*)$ beschrieben werden. Die sechste prädiktive Verteilung kann mittels Gleichung (16) ermittelt werden:

$$p(f(x)|D_n, g_k^*) = \int p(f(x)|D_n, g(x))\, p(g(x)|D_n, g_k^*) dg(x) \tag{16}$$

wobei die prädiktive Verteilung $p(f(x)|D_n, g(x))$ durch eine GP-Regression ermittelt werden kann, wobei $p(f(x)|D_n, g(x)) = \mathcal{N}(f(x)|\mu_f, \Sigma_f)$ ist, und

wobei $p(g(x)|D_n, g_k^*)$ eine siebte prädiktive Verteilung ist und mittels einer gestutzten Normalverteilung (truncated normal distribution) oder mittels einer Gauß-Approximation ermittelt werden kann.

Im Folgenden wird das Ermitteln der siebten prädiktiven Verteilung ( $g(x)|D_n, g_k^*$ ) mittels einer Gauß-Approximation beschrieben. Die prädiktive Verteilung $p(g|D_n, g_k^*)$ kann mittels Gleichung (17) ermittelt, beispielsweise approximiert, werden:

$$p(g|D_n, g_k^*) \propto p(g|D_n) \prod_{i=1}^{n} \mathbb{1}_{\{x_i | g(x_i) \le g_k^*\}} \approx \mathcal{N}(g|\mu_1, \Sigma_1) \tag{17}$$

wobei

$$\mathbb{1}_{\{\cdot\}}$$

eine Indikatorfunktion ist.

Ein Ermitteln der prädiktiven Verteilung $p(g(x)|g, D_n)$ mittels einer GP-Regression und dem Einsetzen in Gleichung (17) führt zu Gleichung (18):

$$p_0(g(x)|D_n, g_k^*) = \int p(g(x)|g, D_n)\, p(g|D_n, g_k^*) dg \approx \mathcal{N}(g(x)|m_0(x), v_0(x)) \tag{18}$$

und das Einfügen der Beschränkung $g(x) \le g_k^*$ in Gleichung (18) führt zu Gleichung (19):

$$p(g(x)|D_n, g_k^*) \propto p_0(g(x)|D_n, g_k^*)\, \mathbb{1}_{\{x | g(x) \le g_k^*\}} \approx \mathcal{N}(g(x)|\widehat{m}(x), \hat{v}(x)) \tag{19}$$

wobei $\widehat{m}(x) = m_0(x) - \sqrt{v_0(x)r}$ und $\hat{v}(x) = v_0(x) - v_0(x)r(r + \beta)$ ist, wobei $\beta = (g_k^* - m_0(x))/\sqrt{v_0(x)}$ und $r = \varphi(\beta)/\Phi(\beta)$ ist, und wobei $\varphi(\blacksquare)$ die Wahrscheinlichkeitsdichtefunktion und $\Phi(\blacksquare)$ die kumulative Dichtefunktion einer Standardnormalverteilung sind.

Basierend darauf kann die sechste prädiktive Verteilung $p(f(x)|D_n, g_k^*)$ mittels Gleichung (20) ermittelt, beispielsweise approximiert, werden:

$$p(f(x)|D_n, g_k^*) \approx \mathcal{N}(f(x)|\hat{m}_k(x), \hat{v}_k(x)) \tag{20}$$

Die mittels Expectation Propagation (EP) ermittelte Akquisitionsfunktion 302 ($\alpha_{NES\text{-}EP}(x)$) kann durch Gleichung (21) beschrieben werden, wobei der Rauschanteil $\varepsilon$ durch die Varianz $\sigma_\epsilon^2$ berücksichtigt wird:

$$\alpha_{NES-EP}(x) = \frac{1}{2}\left[\log\left(v_f(x|D_n) + \sigma_\epsilon^2\right) - \frac{1}{K}\sum_{g_k^* \in G^*} \log(\hat{v}_k(x) + \sigma_\epsilon^2)\right] \tag{21}$$

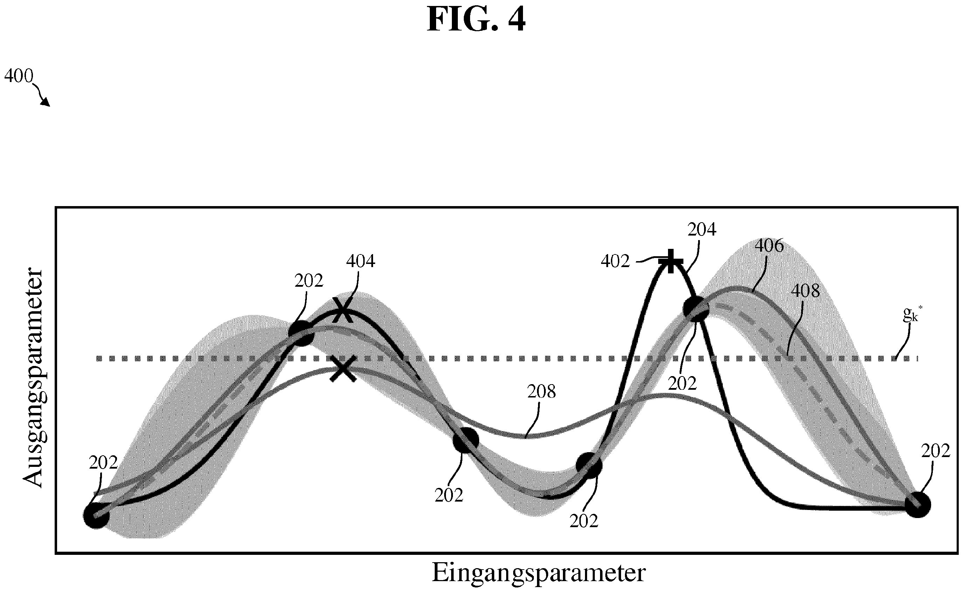

**FIG. 4** stellt eine beispielhafte Verteilung eines ersten statistischen Modells 204 dar. Das dargestellte beispielhafte erste statistische Modell 204 kann einen physikalischen oder chemischen Prozess zu den bekannten Messpunkten 202 beschreiben und kann ein globales Maximum 402 und ein lokales Maximum 404, zum Beispiel ein robustes Optimum, aufweisen. Für das erste statistische Modell 204 kann eine beispielhafte (prädiktive) Verteilung ermittelt werden. Für das erste statistische Modell 204 kann zum Beispiel die fünfte prädiktive Verteilung 406, d.h. $p(f(x)|D_n)$, ermittelt werden, wobei das erste statistische Modell 204 auf die bekannten Messpunkte 202 ($D_n$) konditioniert wird. Beispielsweise kann ferner die sechste prädiktive Verteilung 408, d.h. $p(f(x)|D_n, g_k^*)$, ermittelt werden, indem das erste statistische Modell 204 auf die bekannten Messpunkte 202 ($D_n$) und auf die mehreren approximierten Maximalwerte ($g_k^*$) konditioniert wird.

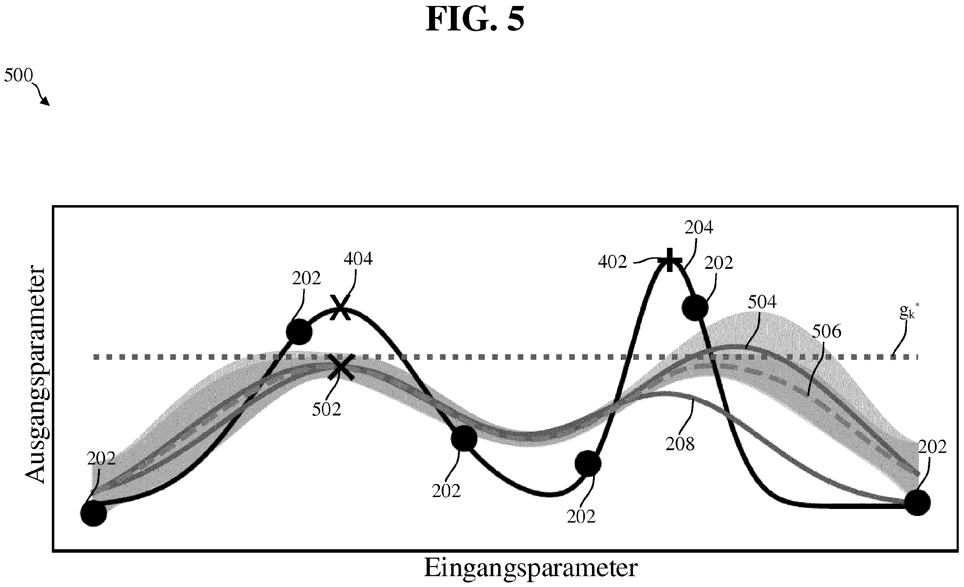

**FIG. 5** stellt eine beispielhafte Verteilung eines zweiten statistischen Modells 208 dar. Das dargestellte beispielhafte zweite statistische Modell 208 unter Verwendung des beispielhaften ersten statistischen Modells 204 und der Veränderung der Eingangsparameterwerte 206 ermittelt worden sein. Das zweite statistische Modell 208 kann ein globales Maximum 502 aufweisen. Der dem globalen Maximum 502 des zweiten statistischen Modells 208 zugeordnete Eingangsparameterwert kann der Eingangsparameterwert des neuen Messpunktes 210 sein. Der dem globalen Maximum 502 des zweiten statistischen Modells 208 zugeordnete Eingangsparameterwert kann dem lokalen Maximum 404 des ersten statistischen Modells 204 zugeordneten Eingangsparameterwert entsprechen, wobei das lokale Maximum ein robustes Optimum sein kann. Für das zweite statistische Modell 208 kann eine beispielhafte (prädiktive) Verteilung ermittelt werden. Für das zweite statistische Modell 208 kann zum Beispiel die vierte prädiktive Verteilung 504, d.h. $p(g(x)|D_n)$, ermittelt werden, wobei das zweite statistische Modell 208 auf die bekannten Messpunkte 202 ($D_n$) konditioniert wird. Beispielsweise kann ferner die siebte prädiktive Verteilung 506, d.h. $p(g(x)|D_n, g_k^*)$, ermittelt werden, indem das zweite statistische Modell 208 auf die bekannten Messpunkte 202 ($D_n$) und auf die mehreren approximierten Maximalwerte ($g_k^*$) des Ausgangsparameters konditioniert wird.

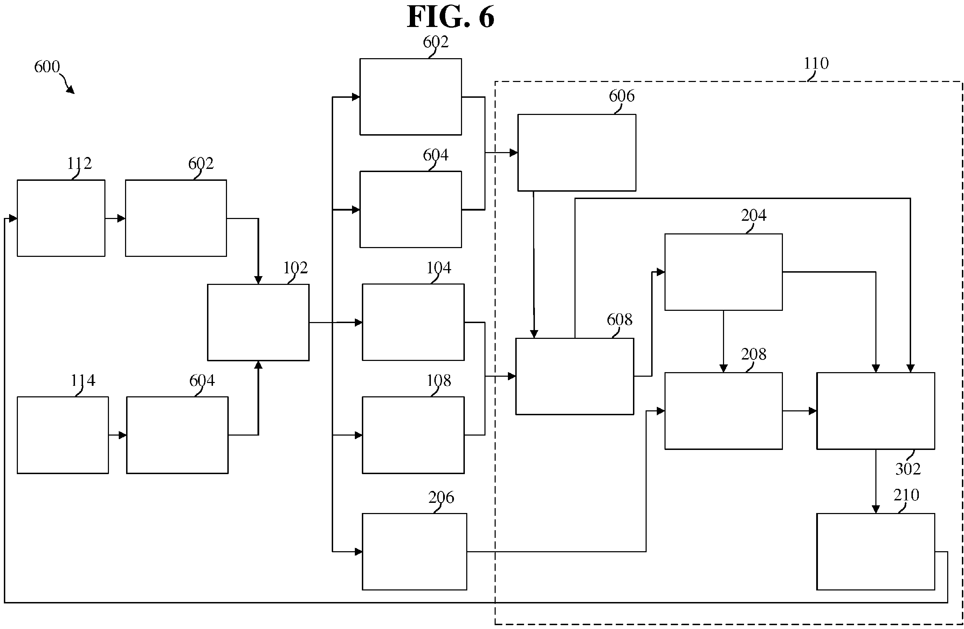

**FIG. 6** stellt ein Verarbeitungssystem 600 zum Adaptieren eines statistischen Modells gemäß verschiedenen Ausführungsformen dar. Das Verarbeitungssystem 600 kann im Wesentlichen dem Verarbeitungssystem 300 entsprechen, wobei das Verarbeitungssystem 600 ferner eingerichtet ist, um für den Eingangsparameterwert des neuen Messpunktes 210 einen zugeordneten Ausgangsparameterwert zu messen, d.h. den neuen Messpunkt 210 zu messen.

Der erste Sensor 112 kann eingerichtet sein, um unter Verwendung des Eingangsparameterwertes des neuen Messpunktes 210 einen neuen Eingangsparameterwert 602 bereitzustellen, wobei der neue Eingangsparameterwert 602 im Wesentlichen dem Eingangsparameterwert des neuen Messpunktes 210 entsprechen kann. Anders ausgedrückt, gibt in diesem Fall der Eingangsparameterwert des neuen Messpunktes 210 an, für welchen Eingangsparameterwert ein neuer Eingangsparameterwert 602 detektiert werden soll. Das heißt, der neue Eingangsparameterwert 602 kann von dem Eingangsparameterwert des neuen Messpunktes 210 aufgrund von Rauschen, Messabweichungen, einer Unsicherheit des Eingangsparameters usw. abweichen. Gemäß verschiedenen Ausführungsformen weist das Verarbeitungssystem 600 ferner eine Steuervorrichtung auf, wobei die Steuervorrichtung den Prozessor 110 aufweisen kann und wobei die Steuervorrichtung eingerichtet sein kann, um einen Prozess derart zu steuern, dass ein durch den ersten Sensor 112 bereitgestellter Eingangsparameterwert im Wesentlichen dem Eingangsparameterwert des neuen Messpunktes 210 entspricht. Gemäß verschiedenen Ausführungsformen stellt der Prozessor 110 den Eingangsparameterwert des neuen Messpunktes 210, zum Beispiel als neuen Eingangsparameterwert 602, der Speichervorrichtung 102 bereit. Das heißt, dass der Eingangsparameterwert des neuen Messpunktes 210 dem neuen Eingangsparameterwert entsprechen kann.

Der zweite Sensor 114 kann eingerichtet sein, einen dem neuen Eingangsparameterwert 602 zugeordneten neuen Ausgangsparameterwert 604 bereitzustellen. Die Speichervorrichtung 102 kann eingerichtet sein, den neuen Eingangsparameterwert 602 und den neuen Ausgangsparameterwert 604 zu speichern. Das Verarbeitungssystem 600 kann ferner den mindestens einen Prozessor 110 aufweisen. Der Prozessor 110 kann eingerichtet sein, um einen gemessenen neuen Messpunkt 606 zu ermitteln, wobei der gemessene neue Messpunkt 606 den neuen Eingangsparameterwert 602 und den dem neuen Eingangsparameterwert 602 zugeordneten neuen Ausgangsparameterwert 604 aufweist. Der Prozessor 110 kann eingerichtet sein, um die bekannten Messpunkte 202 mit dem gemessenen neuen Messpunkt 606 zu aktualisieren. Das heißt, der Prozessor 110 kann eingerichtet sein, aktualisierte bekannte Messpunkte 608 zu ermitteln, wobei die aktualisierten bekannten Messpunkte 608 die bekannten Messpunkte 202 und den gemessenen neuen Messpunkt 606 aufweisen. Das heißt, die aktualisierten bekannten Messpunkte 608 ($D_{n+1}$) können durch $D_{n+1} = \{(x_i, y_i)\}_{i=1:n+1}$ beschrieben werden.

Der Prozessor 110 kann ferner eingerichtet sein, das erste statistische Modell 204 unter Verwendung des gemessenen neuen Messpunktes 606 zu adaptieren. Das Adaptieren des ersten statistischen Modells 204 kann das Ermitteln des ersten statistischen Modells 204 unter Verwendung der aktualisierten bekannten Messpunkte 608 ($D_{n+1}$) aufweisen. Das Ermitteln des ersten statistischen Modells 204 für die aktualisierten bekannten Messpunkte 608 kann im Wesentlichen dem Ermitteln des ersten statistischen Modells 204 für die bekannten Messpunkte 202 entsprechen, wobei die bekannten Messpunkte 202 durch die aktualisierten bekannten Messpunkte 608 ersetzt werden. Anders ausgedrückt, kann das adaptierte erste statistische Modell 204 den physikalischen oder chemischen Prozess zu den bekannten Messpunkten 202 und den gemessenen neuen Messpunkt 210 beschreiben.

Der Prozessor 110 kann eingerichtet sein, das zweite statistische Modell 208 unter Verwendung des adaptierten ersten statistischen Modells 204 und der Veränderung der Eingangsparameterwerte 206 zu ermitteln, wobei das Ermitteln des adaptierten zweiten statistischen Modells 208 im Wesentlichen dem Ermitteln des zweiten statistischen Modells 208 gemäß dem Verarbeitungssystem 300 entsprechen kann. Anders ausgedrückt, kann das adaptierte zweite statistische Modell 208 ermittelt werden, indem das zweite statistische Modell 208 für das adaptierte erste statistische Modell 204 ermittelt wird. Noch anders ausgedrückt werden das erste statistische Modell 204, das zweite statistische Modell 208 und die jeweiligen prädiktiven Verteilungen auf die aktualisierten bekannten Messpunkte 608 konditioniert.

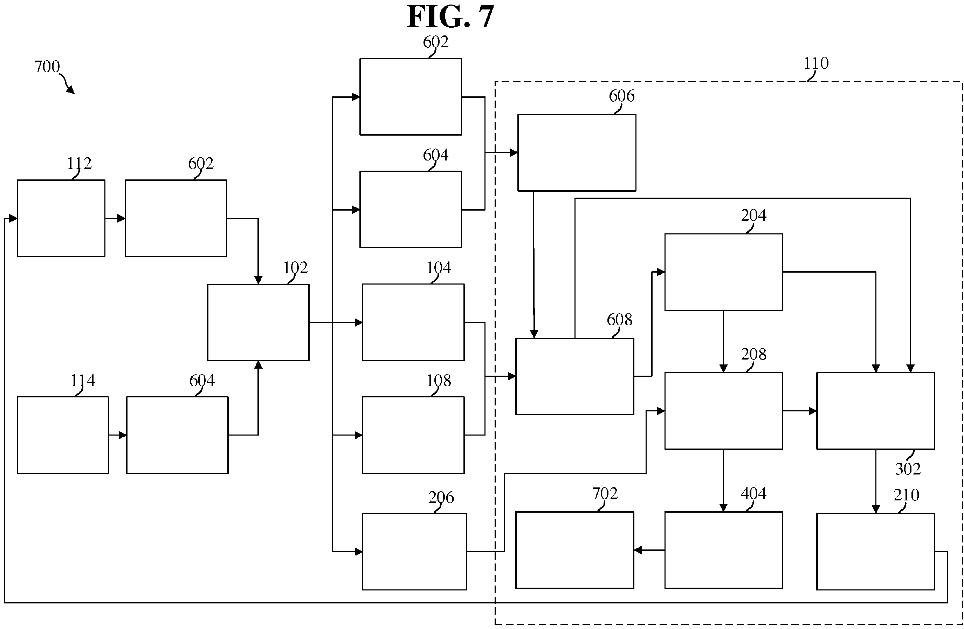

**FIG. 7** stellt ein Verarbeitungssystem 700 zum Ermitteln eines robusten Optimums eines physikalischen oder chemischen Prozesses gemäß verschiedenen Ausführungsformen dar. Das Verarbeitungssystem 700 kann im Wesentlichen dem Verarbeitungssystem 600 entsprechen, wobei der Prozessor 110 ferner eingerichtet ist, um ein robustes Optimum 404 des ersten statistischen Modells 204 zu ermitteln. Der Prozessor 110 kann eingerichtet sein, um ein globales Maximum 502 des adaptierten zweiten statistischen Modells 208 zu ermitteln. Der Prozessor 110 kann ferner eingerichtet sein, ein robustes Optimum 404 des ersten statistischen Modells 204, wobei der Eingangsparameterwert des globalen Maximums 502 des adaptierten zweiten statistischen Modells 208 dem Eingangsparameterwert des robusten Optimums 404 entspricht.

Der Prozessor 110 kann ferner eingerichtet sein, ein Prozessfenster 702 des physikalischen oder chemischen Prozesses zu ermitteln. Das Prozessfenster 702 kann unter Verwendung des robusten Optimums 404 und der Veränderung der Eingangsparameterwerte 206 ermittelt werden. Die Veränderung der Eingangsparameterwerte 206 kann durch eine Verteilung, wie beispielsweise eine Normalverteilung beschrieben sein. Für den Fall, dass die Veränderung der Eingangsparameterwerte 206 durch einen absoluten Wert gegeben ist, kann das Prozessfenster 702 ($x_{Prozessfenster}$) kann durch $x_{neu} - \xi \leq x_{Prozessfenster} \leq x_{neu} + \%$ gegeben sein, wobei $x_{neu}$ der Eingangsparameterwert des robusten Optimums und % die Veränderung der Eingangsparameterwerte 206 ist. Das heißt, das Prozessfenster 702 kann alle Eingangsparameterwerte, d.h. alle $x_{Prozessfenster}$ aufweisen, die innerhalb der Veränderung der Eingangsparameterwerte 206 von dem Eingangsparameterwert des robusten Optimums liegen.

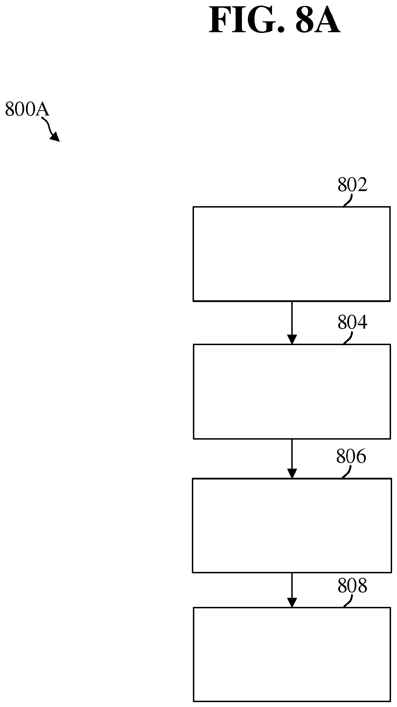

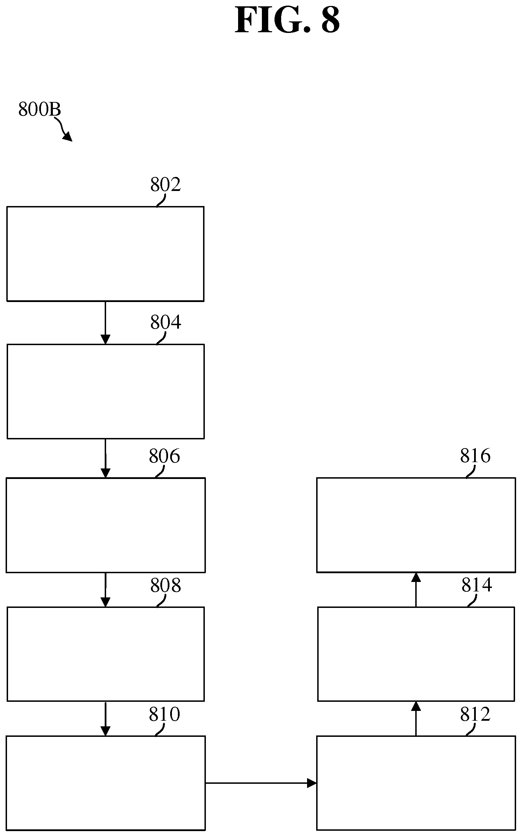

**FIG. 8A** stellt ein Verfahren 800A zum Auswählen eines neuen Messpunktes eines physikalischen oder chemischen Prozesses gemäß verschiedenen Ausführungsformen dar. Das Verfahren 800A kann das Bereitstellen, wie beispielsweise ein Messen, bekannter Messpunkte 202 aufweisen (in 802). Jeder Messpunkt der bekannten Messpunkte 202 kann einen Eingangsparameterwert und einen dem Eingangsparameterwert zugeordneten Ausgangsparameterwert aufweisen. Das Verfahren 800A kann das Ermitteln eines ersten statistischen Modells 204 aufweisen (in 804). Das erste statistische Modell 204 kann einen physikalischen oder chemischen Prozess zu den bekannten Messpunkten 202 beschreiben. Das Verfahren 800A kann das Ermitteln eines zweiten statistischen Modells 208 aufweisen (in 806). Das zweite statistische Modell 208 kann unter Verwendung des ersten statistischen Modells 204 und einer Veränderung der Eingangsparameterwerte 206 ermittelt werden. Das Verfahren 800A kann ferner das Auswählen eines neuen Messpunktes 210 aufweisen (in 808). Der neue Messpunkt 210 kann unter Verwendung des ersten statistischen Modells 204 und der bekannten Messpunkte 202 mit der Konditionierung auf einen Maximalwert des Ausgangsparameters des zweiten statistischen Modells 208 ermittelt werden. Der neue Messpunkt 210 kann einen Eingangsparameterwert aufweisen, für den durch Ermitteln des zugeordneten Ausgangsparameterwertes ein robustes Optimum ermittelt werden kann.

**FIG. 8B** stellt ein Verfahren 800B zum Ermitteln eines robusten Optimums eines physikalischen oder chemischen Prozesses gemäß verschiedenen Ausführungsformen dar. Das Verfahren 800B kann im Wesentlichen dem Verfahren 800A entsprechen, wobei das Verfahren 800B ferner das Messen des dem Eingangsparameterwert des neuen Messpunktes 210 zugeordneten Ausgangsparameterwertes aufweisen kann (810). Der Eingangsparameterwert des neuen Messpunktes 210 und der zugeordnete gemessene Ausgangsparameterwert können einen gemessenen neuen Messpunkt 606 bilden. Das Verfahren 800B kann das Adaptieren des ersten statistischen Modells 204 aufweisen (in 812). Das adaptierte erste statistische Modell 204 kann unter Verwendung des gemessenen neuen Messpunktes 606 ermittelt werden. Das adaptierte erste statistische Modell 204 kann den physikalischen oder chemischen Prozess zu den bekannten Messpunkten 202 und dem gemessenen neuen Messpunkt 606 beschreiben. Das Verfahren 800B kann das Adaptieren des zweiten statistischen Modells 208 aufweisen (in 814). Das adaptierte zweite statistische Modell 208 kann für das adaptierte erste statistische Modell 204 ermittelt werden. Das Verfahren 800B kann das Ermitteln eines robusten Optimums 404 des adaptierten ersten statistischen Modells 204 aufweisen (in 816). Das Ermitteln des robusten Optimums 404 kann das Ermitteln eines globalen Maximums 502 des adaptierten zweiten statistischen Modells 208 aufweisen, wobei der Eingangsparameterwert des globalen Maximums 502 des adaptierten zweiten statistischen Modells 208 dem Eingangsparameterwert des robusten Optimums 404 des ersten statistischen Modells entspricht. Unter Verwendung des robusten Optimums 404 und der Veränderung der Eingangsparameterwerte 206 kann ein Prozessfenster 702 des physikalischen oder chemischen Prozesses ermittelt werden. Das Verfahren 800B kann ferner das Steuern des physikalischen oder chemischen Prozesses aufweisen, wobei der Eingangsparameterwert des physikalischen oder chemischen Prozesses in Abhängigkeit von dem Eingangsparameterwert des robusten Optimums gewählt werden kann und wobei der Eingangsparameterwert dem Eingangsparameterwert des robusten Optimums entsprechen kann.

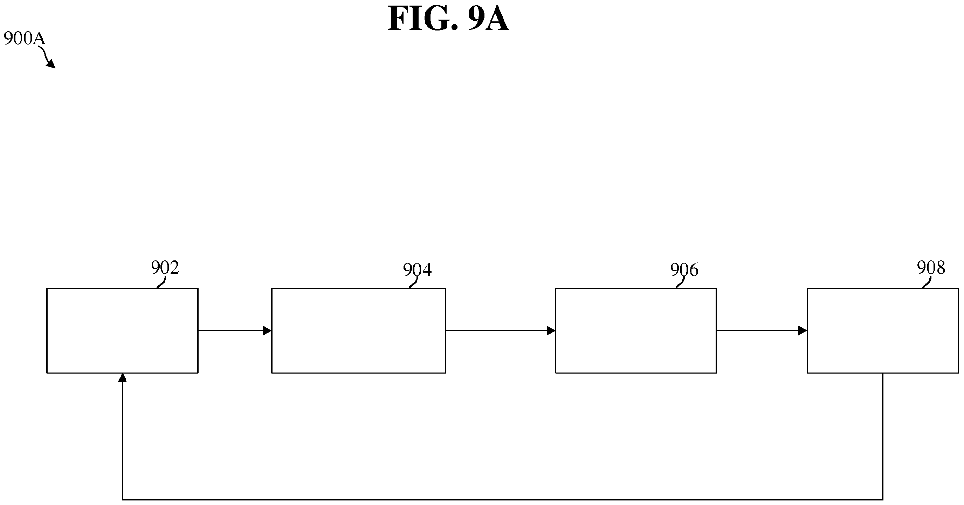

FIG. 9A stellt ein System 900A zum Ermitteln eines robusten Optimums eines physikalischen oder chemischen Prozesses gemäß verschiedenen Ausführungsformen dar. Das System 900A kann eine erste Vorrichtung 902 aufweisen, an der ein physikalischer Prozess und/oder ein chemischer Prozess ausgeführt wird. Die erste Vorrichtung 902 kann zum Beispiel eine Produktionsvorrichtung zum Herstellen von Produkten oder eine Bearbeitungsvorrichtung zum Bearbeiten von Werkstücken (zum Beispiel eine Bohrvorrichtung oder eine Fräsvorrichtung), eine Robotervorrichtung die beispielsweise Bewegungen ausführt, eine Vorrichtung zum Wirkstoff-Design beispielsweise eines Arzneimittels, eine Vorrichtung zum Design eines Systems, beispielsweise für die Luft- und Raumfahrt, usw. sein. Das System 900A kann ferner einen Sensor 904 aufweisen. Der Sensor 904 kann eingerichtet sein, um mehrere Ausgangsparameterwerte 108 zu detektieren, die bereitgestellten mehreren Eingangsparameterwerten zugeordnet sind. Der Sensor 904 kann eingerichtet sein, um die mehreren Ausgangsparameterwerte 108 während einem durch die erste Vorrichtung 902 ausgeführten physikalischen oder chemischen Prozess (in-situ) zu detektieren. Gemäß verschiedenen Ausführungsformen ist der Sensor 904 eingerichtet, um die mehreren Ausgangsparameterwerte 108 nach einem durch die erste Vorrichtung 902 ausgeführten physikalischen oder chemischen Prozess (exsitu) zu detektieren. Anders ausgedrückt, können die mehreren Eingangsparameterwerte 104 während dem physikalischen oder chemischen Prozess eingestellt oder von einem zusätzlichen Sensor detektiert werden und mehrere Ausgangsparameterwerte 108 können nach dem physikalischen oder chemischen Prozess detektiert werden, sodass sich die Detektion der mehreren Ausgangsparameterwerte 108 zeitlich unterscheidet. Zum Beispiel kann die erste Vorrichtung 902 ein Ofen zum Härten eines Werkstücks sein, ein Eingangsparameterwert der mehreren Eingangsparameterwerte 104 kann eine im Ofen eingestellte oder gemessene Temperatur sein, und ein dem Eingangsparameterwert zugeordneter Ausgangsparameterwert der mehreren Ausgangsparameterwerte 108 kann eine im Anschluss an das Härten des Werkstücks detektierte Härte des Werkstücks sein.

Das System 900A kann ferner eine zweite Vorrichtung 906 aufweisen. Die zweite Vorrichtung 906 kann eingerichtet sein, das Verfahren 800A und/oder das Verfahren 800B durchzuführen, wobei der Sensor 904 eingerichtet sein kann, der zweiten Vorrichtung 906 die mehreren Eingangsparameterwerten 104 zugeordneten mehreren Ausgangsparameterwerte 108 bereitzustellen. Die zweite Vorrichtung 906 kann eingerichtet sein, einen neuen Messpunkt gemäß dem Verfahren 800A auszuwählen. Das System 900A kann ferner eine Steuervorrichtung 908 aufweisen. Die Steuervorrichtung 908 kann eingerichtet sein, um den von der zweiten Vorrichtung 908 ausgewählten neuen Messpunkt zu empfangen und um die erste Vorrichtung 902 derart zu steuern, dass ein eingestellter Eingangsparameterwert dem Eingangsparameterwert des ausgewählten neuen Messpunktes 210 entspricht. Der Sensor 904 kann eingerichtet sein, den dem eingestellten Eingangsparameterwert zugeordneten Ausgangsparameterwert des neuen Messpunktes 210 zu detektieren und der zweiten Vorrichtung 906 bereitzustellen. Die zweite Vorrichtung 906 kann eingerichtet sein, um das erste statistische Modell 204 und das zweite statistische Modell 208 gemäß dem Verfahren 800B zu adaptieren. Die zweite Vorrichtung 906 kann ferner eingerichtet sein, ein robustes Optimum 404 des ersten statistischen Modells 204 gemäß dem Verfahren 800B zu ermitteln.

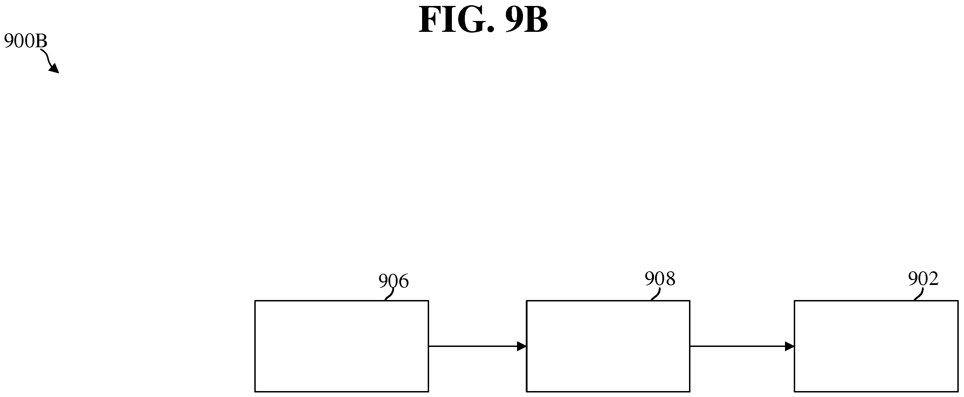

FIG. 9B stellt ein System 900B dar, das ein gemäß verschiedenen Ausführungsformen adaptiertes statistisches Modell verwendet. Das System 900B kann die zweite Vorrichtung 908 aufweisen, die ein gemäß dem Verfahren 800B adaptiertes erstes statistisches Modell 204 und adaptiertes zweites statistisches Modell 208 aufweisen kann. Die zweite Vorrichtung 906 kann eingerichtet sein, unter Verwendung des ersten statistischen Modells 204 und des zweiten statistischen Modells 208 ein robustes Optimum 404 des ersten statistischen Modells 204 zu ermitteln. Das System 900B kann ferner die Steuervorrichtung 908 aufweisen. Die Steuervorrichtung 908 kann eingerichtet sein, das ermittelte robuste Optimum 404 von der zweiten Vorrichtung 906 zu empfangen. Das System 900B kann ferner die erste Vorrichtung 902 aufweisen, an der ein physikalischer Prozess und/oder ein chemischer Prozess ausgeführt wird. Die Steuervorrichtung 908 kann eingerichtet sein, die erste Vorrichtung derart zu steuern, dass ein an der ersten Vorrichtung eingestellter Eingangsparameterwert in Abhängigkeit des Eingangsparameterwertes des robusten Optimums 404 gewählt wird, wobei der eingestellte Eingangsparameterwert dem Eingangsparameterwert des robusten Optimums 404 entsprechen kann.

**Patentansprüche**

1. Verfahren zum Ermitteln eines robusten Optimums eines physikalischen oder chemischen Prozesses gemäß einem Bayes-Optimierungsverfahren, wobei jeder Messpunkt von bekannten Messpunkten einen Eingangsparameterwert eines physikalischen oder chemischen Prozesses und einen dem Eingangsparameterwert zugeordneten gemessenen Ausgangsparameterwert aufweist, und wobei ein erstes statistisches Modell den Zusammenhang zwischen den Eingangsparameterwerten und den Ausgangsparameterwerten des physikalischen oder chemischen Prozesses beschreibt, das Verfahren aufweisend:

   • Ermitteln eines zweiten statistischen Modells für das erste statistische Modell, wobei das zweite statistische Modell eine Robustheit für die Ausgangsparameterwerte des physikalischen oder chemischen Prozesses bezüglich einer Veränderung der Eingangsparameterwerte beschreibt;
   • wobei ein neuer Messpunkt derart ausgewählt wird, dass eine Differenz aus

      ◦ einer Entropie des durch die bekannten Messpunkte beschriebenen ersten statistischen Modells für den physikalischen oder chemischen Prozess an dem neuen Messpunkt, und
      ◦ einem Erwartungswert einer Entropie des durch die bekannten Messpunkte beschriebenen ersten statistischen Modells an dem neuen Messpunkt mit einer Konditionierung auf einen angenommenen Maximalwert des Ausgangsparameters des zweiten statistischen Modells an dem zu ermittelnden robusten Optimum des physikalischen oder chemischen Prozesses,
      im Wesentlichen maximal ist oder in einem vorgegebenen Umgebungsbereich des Maximums liegt;

   • Messen des dem Eingangsparameterwert des neuen Messpunktes zugeordneten Ausgangsparameterwertes;
   • Adaptieren des ersten statistischen Modells unter Verwendung des neuen Messpunktes;
   • Ermitteln des robusten Optimums des adaptierten ersten statistischen Modells; und
   • wobei das adaptierte erste statistische Modell den physikalischen oder chemischen Prozess zu den bekannten Messpunkten und dem neuen Messpunkt beschreibt;
   • wobei das Ermitteln des robusten Optimums des ersten statistischen Modells aufweist:

      ◦ Ermitteln eines adaptierten zweiten statistischen Modells für das adaptierte erste statistische Modell; und
      ◦ Ermitteln eines globalen Maximums des adaptierten zweiten statistischen Modells, wobei der Eingangsparameterwert des globalen Maximums des zweiten statistischen Modells dem Eingangsparameterwert des robusten Optimums des ersten statistischen Modells entspricht; und

   • Steuern des physikalischen oder chemischen Prozesses, wobei der Eingangsparameterwert des physikalischen oder chemischen Prozesses in Abhängigkeit von dem Eingangsparameterwert des robusten Optimums gewählt wird.

2. Verfahren nach Anspruch 1, ferner aufweisend:
   Ermitteln eines Prozessfensters des physikalischen oder chemischen Prozesses unter Verwendung des robusten Optimums und der Veränderung der Eingangsparameterwerte.

3. Vorrichtung, die eingerichtet ist, das Verfahren nach einem der Ansprüche 1 oder 2 auszuführen.

4. Produktionssystem, aufweisend:

   eine Vorrichtung nach Anspruch 3;
   eine Produktionsvorrichtung, die eingerichtet ist, ein Produkt herzustellen;
   zumindest einen Sensor, der eingerichtet ist, der Vorrichtung Eingangsparameterwerten zugeordnete Ausgangsparameterwerte bereitzustellen; und
   eine Steuervorrichtung, die eingerichtet ist, die Produktionsvorrichtung derart zu steuern, dass ein Eingangsparameterwert in Abhängigkeit des Eingangsparameterwertes des robusten Optimums gewählt wird.

**5.** Bearbeitungssystem, aufweisend:

eine Vorrichtung nach Anspruch 3;
eine Bearbeitungsvorrichtung, die eingerichtet ist, ein Werkstück zu bearbeiten;
zumindest einen Sensor, der eingerichtet ist, der Vorrichtung Eingangsparameterwerten zugeordnete Ausgangsparameterwerte bereitzustellen; und
eine Steuervorrichtung, die eingerichtet ist, die Bearbeitungsvorrichtung derart zu steuern, dass ein Eingangsparameterwert in Abhängigkeit des Eingangsparameterwertes des robusten Optimums gewählt wird.

**6.** Robotersystem, aufweisend:

eine Vorrichtung nach Anspruch 3;
eine Robotervorrichtung, die eingerichtet ist, eine Bewegung auszuführen;
zumindest einen Sensor, der eingerichtet ist, der Vorrichtung Eingangsparameterwerten zugeordnete Ausgangsparameterwerte bereitzustellen; und
eine Steuervorrichtung, die eingerichtet ist, die Robotervorrichtung derart zu steuern, dass ein Eingangsparameterwert in Abhängigkeit des Eingangsparameterwertes des robusten Optimums gewählt wird.

**Claims**

**1.** Method for determining a robust optimum of a physical or chemical process according to a Bayesian optimization method, wherein each measurement point of known measurement points has an input parameter value of a physical or chemical process and a measured output parameter value assigned to the input parameter value, and wherein a first statistical model describes the relationship between the input parameter values and the output parameter values of the physical or chemical process, the method comprising:

• determining a second statistical model for the first statistical model, wherein the second statistical model describes a robustness for the output parameter values of the physical or chemical process in relation to a change in the input parameter values;
• wherein a new measurement point is selected in such a way that a difference between

◦ an entropy of the first statistical model described by the known measurement points for the physical or chemical process at the new measurement point and
◦ an expected value of an entropy of the first statistical model described by the known measurement points at the new measurement point with a conditioning on an assumed maximum value of the output parameter of the second statistical model at the robust optimum to be determined of the physical or chemical process is substantially maximal or lies in a predefined range surrounding the maximum;

• measuring the output parameter value assigned to the input parameter value of the new measurement point;
• adapting the first statistical model using the new measurement point;
• determining the robust optimum of the adapted first statistical model; and
• wherein the adapted first statistical model describes the physical or chemical process with respect to the known measurement points and the new measurement point;
• wherein determining the robust optimum of the first statistical model comprises:

◦ determining an adapted second statistical model for the adapted first statistical model; and
◦ determining a global maximum of the adapted second statistical model, wherein the input parameter value of the global maximum of the second statistical model corresponds to the input parameter value of the robust optimum of the first statistical model; and

• controlling the physical or chemical process, wherein the input parameter value of the physical or chemical process is chosen as a function of the input parameter value of the robust optimum.

**2.** Method according to Claim 1, furthermore comprising: determining a process window of the physical or chemical process using the robust optimum and the change in the input parameter values.

3. Device configured to carry out the method according to either of Claims 1 and 2.

4. Production system, comprising:

> a device according to Claim 3;
> a production device configured to produce a product;
> at least one sensor configured to provide the device with output parameter values assigned to input parameter values; and
> a control device configured to control the production device in such a way that an input parameter value is chosen as a function of the input parameter value of the robust optimum.

5. Processing system, comprising:

> a device according to Claim 3;
> a processing device configured to process a workpiece;
> at least one sensor configured to provide the device with output parameter values assigned to input parameter values; and
> a control device configured to control the processing device in such a way that an input parameter value is chosen as a function of the input parameter value of the robust optimum.

6. Robotic system, comprising:

> a device according to Claim 3;
> a robotic device configured to carry out a movement;
> at least one sensor configured to provide the device with output parameter values assigned to input parameter values; and
> a control device configured to control the robotic device in such a way that an input parameter value is chosen as a function of the input parameter value of the robust optimum.

**Revendications**

1. Procédé permettant d'établir un optimum fiable d'un processus physique ou chimique selon un procédé d'optimisation de Bayes, dans lequel chaque point de mesure parmi des points de mesure connus présente une valeur de paramètre d'entrée d'un processus physique ou chimique et une valeur de paramètre de sortie mesurée associée à la valeur de paramètre d'entrée, et dans lequel un premier modèle statistique décrit la relation entre les valeurs de paramètre d'entrée et les valeurs de paramètre de sortie du processus physique ou chimique, le procédé présentant les étapes consistant à :

> • établir un deuxième modèle statistique pour le premier modèle statistique, le deuxième modèle statistique décrivant une fiabilité pour les valeurs de paramètre de sortie du processus physique ou chimique concernant une variation des valeurs de paramètre d'entrée ;
> • dans lequel un nouveau point de mesure est sélectionné de telle sorte qu'une différence entre
>
> > ∘ une entropie du premier modèle statistique décrit par les points de mesure connus pour le processus physique ou chimique au nouveau point de mesure, et
> > ∘ une valeur attendue d'une entropie du premier modèle statistique décrit par les points de mesure connus au nouveau point de mesure, avec un conditionnement à une valeur maximale supposée du paramètre de sortie du deuxième modèle statistique au niveau de l'optimum fiable à établir du processus physique ou chimique, est substantiellement maximale ou se situe dans une plage environnante prédéfinie du maximum ;
>
> • mesurer la valeur de paramètre de sortie associée à la valeur de paramètre d'entrée du nouveau point de mesure ;
> • adapter le premier modèle statistique en utilisant le nouveau point de mesure ;
> • établir l'optimum fiable du premier modèle statistique adapté ; et
> • dans lequel le premier modèle statistique adapté décrit le processus physique ou chimique pour les points de mesure connus et le nouveau point de mesure ;
> • dans lequel l'établissement de l'optimum fiable du premier modèle statistique présente :

∘ l'établissement d'un deuxième modèle statistique adapté pour le premier modèle statistique adapté ; et
∘ l'établissement d'un maximum global du deuxième modèle statistique adapté, dans lequel la valeur de paramètre d'entrée du maximum global du deuxième modèle statistique correspond à la valeur de paramètre d'entrée de l'optimum fiable du premier modèle statistique ; et

• commander le processus physique ou chimique, dans lequel la valeur de paramètre d'entrée du processus physique ou chimique est choisie en fonction de la valeur de paramètre d'entrée de l'optimum fiable.

2. Procédé selon la revendication 1, présentant en outre :
l'établissement d'une fenêtre de processus du processus physique ou chimique en utilisant l'optimum fiable et la variation des valeurs de paramètre d'entrée.

3. Dispositif, qui est conçu pour exécuter le procédé selon l'une quelconque des revendications 1 ou 2.

4. Système de production, présentant :

un dispositif selon la revendication 3 ;
un dispositif de production qui est conçu pour fabriquer un produit :

au moins un capteur qui est conçu pour fournir au dispositif des valeurs de paramètre de sortie associées à des valeurs de paramètre d'entrée ; et
un dispositif de commande qui est conçu pour commander le dispositif de production de telle sorte qu'une valeur de paramètre d'entrée soit choisie en fonction de la valeur de paramètre d'entrée de l'optimum fiable.

5. Système d'usinage, présentant :

un dispositif selon la revendication 3 ;
un dispositif d'usinage qui est conçu pour usiner une pièce à travailler ;
au moins un capteur qui est conçu pour fournir au dispositif des valeurs de paramètre de sortie associées à des valeurs de paramètre d'entrée ; et
un dispositif de commande qui est conçu pour commander le dispositif d'usinage de telle sorte qu'une valeur de paramètre d'entrée soit choisie en fonction de la valeur de paramètre d'entrée de l'optimum fiable.

6. Système robotisé, présentant :

un dispositif selon la revendication 3 ;
un dispositif robotisé qui est conçu pour effectuer un mouvement :

au moins un capteur qui est conçu pour fournir au dispositif des valeurs de paramètre de sortie associées à des valeurs de paramètre d'entrée ; et
un dispositif de commande qui est conçu pour commander le dispositif robotisé de telle sorte qu'une valeur de paramètre d'entrée soit choisie en fonction de la valeur de paramètre d'entrée de l'optimum fiable.

EP 3 796 108 B1

# FIG. 1A

100A

# FIG. 1B

EP 3 796 108 B1

# FIG. 2

FIG. 3

# FIG. 4

EP 3 796 108 B1

# FIG. 5

## FIG. 6

EP 3 796 108 B1

# FIG. 7

EP 3 796 108 B1

# FIG. 8A

800A

802

804

806

808

# FIG. 8

800B

EP 3 796 108 B1

# FIG. 9A

900A

# FIG. 9B

900B

EP 3 796 108 B1

906

908

902

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **NOGUEIRA et al.** Unscented Bayesian Optimization for Safe Robot Grasping. *arXiv:1603.02038,* 2016
- **BELAND et al.** Bayesian Optimization Under Uncertainty. *Conference on Neural Information Processing Systems,* 2017
- **BOGUNOVIC et al.** Adversarially Bayesian Optimization with Gaussian Processes. *Conference on Neural Information Processing Systems,* 2018
- **TAKENO S. et al.** Multi-fidelity Bayesian Optimization with Max-value Entropy Search and its parallelization. *arXiv.org:1901.08275v2,* 2019
- **WANG, ZI et al.** Max-Value Entropy Search for Efficient Bayesian Optimization. *arXiv.org:1703.01968v3,* 2017